# EUROPEAN PATENT SPECIFICATION

(11) **EP 2 931 723 B1**
(45) Date of publication and mention of the grant of the patent: **26.08.2020**
(21) Application number: 12889814.5
(22) Date of filing: 11.12.2012
(51) Int. Cl.: C07D 471/08, A61K 31/439, A61K 31/444, A61K 31/4545, A61K 31/4709, A61K 31/496, A61P 31/04

(54) **NEW BICYCLIC COMPOUNDS AND THEIR USE AS ANTIBACTERIAL AGENTS AND -LACTAMASE INHIBITORS**
NEUE BICYCLISCHE VERBINDUNGEN UND DEREN VERWENDUNG ALS ANTIBAKTERIELLE MITTEL UND LACTAMASEINHIBITOREN
NOUVEAUX COMPOSÉS BICYCLIQUES ET LEUR UTILISATION EN TANT QU'AGENTS ANTIBACTÉRIENS ET INHIBITEURS DE -LACTAMASE

(43) Date of publication of application: 21.10.2015
(73) Proprietor: Fedora Pharmaceuticals Inc., Edmonton Alberta T5H 0L9 (CA)
(72) Inventor: MAITI, Samarendra N., Edmonton, Alberta T6J 5G7 (CA); NGUYEN, Dai, Edmonton, Alberta T6L 6J9 (CA); KHAN, Jehangir, Edmonton, Alberta T6R 2W1 (CA); LING, Rong, Edmonton, Alberta T6R 2E3 (CA)
(74) Representative: Dehns
(86) International application number: PCT/IB2012/002675
(87) International publication number: WO 2014/091268

(56) References cited:
- WO-A1-2013/030733
- WO-A1-2013/180197
- WO-A1-2014/033560
- WO-A2-2009/091856
- CA-A1- 2 780 403

## Description

### Field of the invention

The present invention relates to new bicyclic compounds, their preparation and their use as antibacterial agents either alone or in combination with an antibiotic (or plural antibiotics) for the treatment of infections caused by β-lactamase-producing pathogenic bacteria. The compounds of the present invention are β-lactamase inhibiting or non-β-lactamase inhibiting (i.e., some of the compounds of the present invention by themselves would directly inhibit β-lactamase enzymatic function, and others of the compounds of the present invention by themselves would not inhibit some β-lactamase's enzymatic function though they provide synergy and increased potency of activity in combination with antibiotics, e.g., β-lactam antibiotics or non-β-lactam antibiotics). More particularly, the invention is concerned with methods for overcoming antibiotic resistance caused by β-lactamase producing bacteria, the method of preparation of the new compounds, pharmaceutical compositions containing the new compounds, and uses of the compounds.

### Background of the invention

Microbial drug resistance is an unavoidable consequence resulting from abuse and overuse of antimicrobial agents. The rate at which resistance arises among microbial population is often dictated by the extent of use of particular agents in a given environment. Given the degree of popularity of β-lactam (also known as β-lactam) antibiotics, it is not surprising that the prevalence of β-lactamase (also known as β-lactamase) producing strains is increasing worldwide. The most significant known mechanism related to the development of bacterial resistance to the β-lactam antibiotics is the production of class-A, class-B, class-C and class-D β-lactamases that are able to hydrolyze the β-lactam antibiotics resulting in the loss of antibacterial activity. Class-A enzymes preferentially hydrolyze penicillins, class-B enzymes hydrolyze all β-lactams including carbapenems, class-C β-lactamases have a substrate profile favoring cephalosporin hydrolysis, whereas substrate preference for class D β-lactamases include oxacillin and cloxacillin.

The possibility of rescuing individual β-lactam antibiotics by combination with a β-lactamase inhibitor that inactivates the β-lactamase before it can hydrolyze the β-lactam antibiotic has been demonstrated with clinically useful combination between penicillins such as amoxicillin, ampicillin, piperacillin and ticarcillin and β-lactamase inhibitors such as clavulanic acid, sulbactam and tazobactam. Further potential combinations have been described involving various β-lactam antibiotics and newly reported β-lactamase inhibitors including bicyclic monobactams, exomethylene penems and 7-oxo-6-diazabicyclo[3.2.1]octane-2-carboxamide derivatives.

As a result of point mutations and plasmid transfer, the diversity of β-lactamases is increasing constantly. The currently commercial β-lactamase inhibitors are insufficient to counter these new β-lactamases - particularly ineffective against class C producing organisms, newly emerged extended-spectrum β-lactamases (ESBLs) and carbapenemases like IMP, VIM, OXA, KPC, and NDM. Thus there is a need for broad-spectrum β-lactamase inhibitor to combat over 900 β-lactamases including the newly emerged β-lactamases.

Recently, certain diazabicyclic compounds have been disclosed in WO 2009/091856. In addition, a number of diazabicyclic heterocycles have been disclosed in the following patents as β-lactamase inhibitors: US 2003/0199541 A1, US 2004/0157826 A1, US 2004/0097490 A1, US 2005/0020572 A1, US 2006/7112592 B2, US 2006/0189652 A1, US 2008/7439253 B2, US 2009/0018329 A1, EP 1307457 B1, EP 1537117 B1, WO 2002/100860 A2, WO 2002/10172 A1, WO 2003/063864 A2, WO 2004/052891 A1, WO 2004/022563 A1, WO 2008/142285 A1, WO 2009/090320 A1, US 2010/0092443 A1, WO 2010/126820 A2, US 2012/0165533 A1. Other compounds proposed for use in treating bacterial infections are disclosed in WO 2013/180197, WO 2014/033560, WO 2009/091856 and WO 2013/030733.

The compounds of the present invention are new and the structural features are significantly distinct from the compounds described in the patent references cited above.

### Detailed description of the invention

In one aspect, the present invention relates to new, low molecular weight diazabicyclic compounds as claimed in claim 1 (some of which have potent broad-spectrum β-lactamase inhibitory activity and others do not have such activity) that when used in combination with a β-lactam antibiotic or with other non β-lactam antibiotics enhance the activity of the antibiotic against class A, Class B, class C, and class D enzyme producing organisms and thereby enhance the antibacterial properties. The compounds are therefore useful in the treatment of bacterial infections in humans or animals either alone or in combination with β-lactam antibiotics and/or with other non β-lactam antibiotics.

In accordance with the present invention, there are provided (A) new compounds of general formula (I), (B) pharmaceutically acceptable salts of the compounds of formula (I), and (C) pharmaceutically acceptable solvates of the compounds of formula (I) and of their salts: 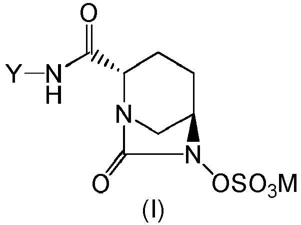 Wherein;
M is hydrogen or a pharmaceutically acceptable salt forming cation,
a "pharmaceutically acceptable salt" refers to a salt of a compound, which salt possesses the desired pharmacological activity of the parent compound,
Y = OR¹ or NR²R³,

In the formula (I), when Y = OR¹, R¹ is a radical selected from any of the following groups:
(1) C₃₋₇ cycloalkyl which is fused with a C₅₋₇ membered saturated heterocycle containing at least one heteroatom which is N. Furthermore, the said bicyclic ring is optionally substituted. Non-limiting examples of such compounds are: 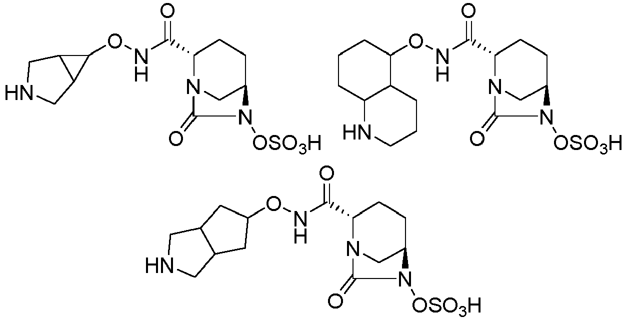
(2) Bridged bicyclic ring system having optionally one heteroatom which is N. Furthermore, the bicyclic ring system is optionally substituted at the free N atom present in the ring. Non-limiting examples of such compounds are: 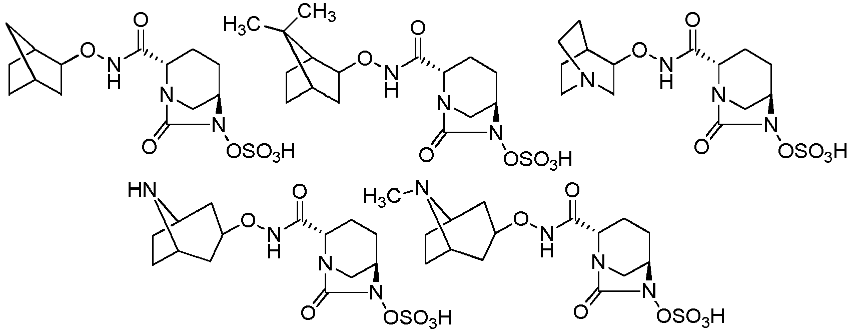
(3) C₅₋₇ membered saturated heterocycles fused to a C₃₋₆ membered ring system through a common carbon atom to form a spiro system containing one heteroatom selected from O and N that is present in the spiro ring and the free N atom present in either ring may optionally take a substituent. Non-limiting examples of such compounds are:

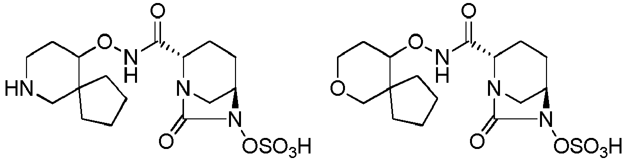

In the above formula (I), when Y = OR¹, several non-limiting examples of the compounds of the present invention are mentioned below:

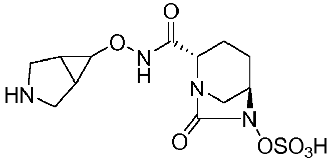

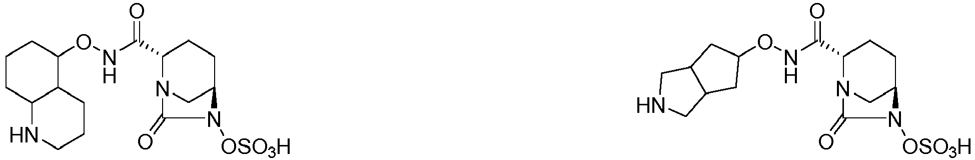

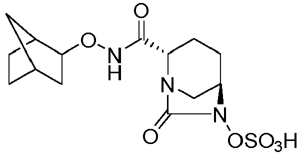

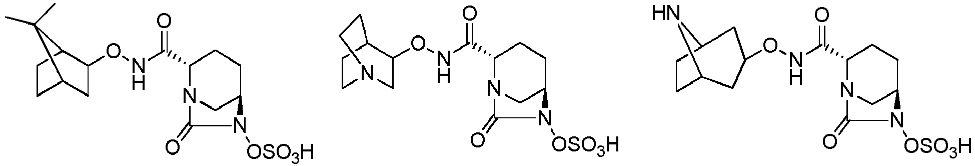

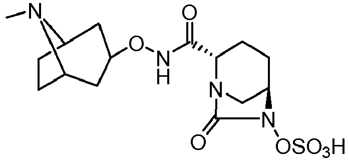

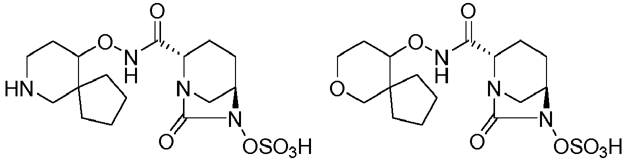

In another embodiment, in the formula (I), Y is NR²R³.

Wherein, R² is hydrogen.

In the formula (I), when Y = NR²R³; R³ is selected from the following groups CH₃SO₂- and NH₂SO₂-. Non-limiting examples of such compounds are:

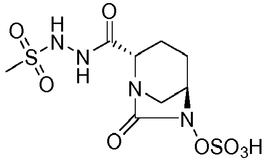

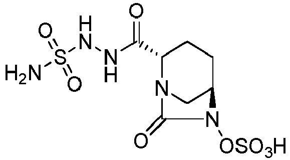

Examples of the groups for forming a pharmaceutically acceptable salt represented by M in the formula (I) include: inorganic base salts, ammonium salts, organic base salts, basic amino acid salts, inorganic acid addition salts, and organic acid addition salts. Inorganic bases that can form the inorganic base salts include alkali metals (e.g., sodium, potassium, and lithium) and alkaline earth metals (e.g., calcium and magnesium). Organic bases that can form the organic base salts include n-propylamine, n-butylamine, cyclohexylamine, benzylamine, octylamine, ethanolamine, diethanolamine, diethylamine, triethylamine, dicyclohexylamine, procaine, choline, N-methylglucamine, morpholine, pyrrolidine, piperidine, N-ethylpiperidine and N-methylmorpholine. Basic amino acids that can form the basic amino acid salts include lysine, arginine, ornithine and histidine. As will be appreciated by one skilled in the art, the compounds of formula (I) containing a basic nitrogen atom are capable of forming acid addition salts. Such salts with pharmaceutically acceptable acids are included in the invention. Examples of such acids are hydrochloric, hydrobromic, phosphoric, sulphuric, citric, oxalic, maleic, fumaric, glycolic, mandelic, tartaric, aspartic, succinic, malic, formic, acetic, trifluoroacetic, methanesulfonic, ethanesulfonic, trifluoromethanesulfonic, benzenesulfonic, p-toluenesulfonic.

Moreover, some compounds of formula (I) when they contain a basic group such as NH, NH₂ may form an inner, zwitterionic salt with OSO₃H; such inner salts are also included in this invention.

For a given compound of the present invention, it is understood that a substituent may be attached at a chiral center of a carbon atom. Therefore the invention includes enantiomers, diastereoisomers or racemates of the compound. By definition 'enantiomers' are a pair of stereoisomers that are non-superimposable mirror images of each other, and 1:1 mixture of a pair of enantiomers is a racemic mixture. By definition, 'diastereoisomers' are stereoisomers that have at least two asymmetric carbon atoms but which are not mirror-images of each other. When a compound of formula (I) is a pure enantiomer the stereochemistry at each chiral carbon may be specified by either R or S.

A variety of protecting groups conventionally used in the β-lactam field to protect a reactive functional group present in the molecule of formula (I) can be used. Protecting group' refers to a group of atoms that when attached to a reactive functional group in a molecule masks, reduces or prevents reactivity of the functional group. Examples of protecting groups can be found in Green et al., "Protective Groups in Organic Chemistry", (Wiley, 2nd, 1991) and Harrison et al., "Compendium of Synthetic Organic Methods," Vols. 1-8 (John Wiley and Sons, 1971-1996). Representative amino protecting groups include, but are not limited to formyl, acetyl, trifluoroacetyl, benzyl, benzyloxycarbonyl (CBZ), *tert*-butoxycarbonyl (Boc), trimethylsilyl (TMS), 2-trimethylsilyl-ethanesulfonyl (SES), trityl and substituted trityl groups, allyloxycarbonyl, 9-fluorenylmethyloxycarbonyl (FMOC), nitro-veratryloxycarbonyl (NVOC). Examples of hydroxy protecting groups include, but are not limited to, those where the hydroxyl group is either acylated or alkylated such as benzyl, and trityl ethers as well as alkyl ethers, tetrahydropyranyl ethers, trialkylsilyl ethers, and allyl ethers.

The term 'optionally substituted' refers to unsubstituted or substituted with one or two of the following substituents each of which is independently selected from:
- Lower alkyl including from one to six carbon atoms in any arrangement, e.g., methyl, ethyl, i-propyl or t-butyl
- Amino
- Substituted amino such as -NHCH₃, -N(CH₃)₂ , -NHCH₂CH₃, -NHPr*ⁱ*, -NHBu^{t}, 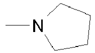
- Alkoxy such as -OCH₃, -OC₂H₅ , -OPrⁱ (i.e., isopropyloxy), -OBu^{t} (i.e., isobutyloxy)
- Hydroxyalkyl such as -CH₂OH, -CH₂CH₂OH
- Halogen such as F, Cl, Br
- Hydroxy
- Carboxy
- Alkoxycarbonyl such as -COOCH₃, -COOC₂H₅, -COOPrⁱ, and -COOBu^{t}
- Haloalkyl such as -CH₂Cl, -CH₂F
- Trifluoromethyl
- Trifluoromethyloxy
- Alkylamine such as -CH₂NH₂, -CH₂CH₂NH₂
- Substituted alkylamine such as -CH₂NHCH₃, -CH₂N(CH₃)₂, -CH₂CH₂NHCH₃, -CH₂CH₂N(CH₃)₂, 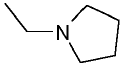
- Carboxamide
- Thiocarboxamide
- Sulfonic acid
- Sulfate
- Acylamino
- Sulfonylamino
- Sulfonamide
- Substituted sulfonamide such as -SO₂NHCH₃, -SO₂NHCH₂CH₃, -SO₂NHPrⁱ, -SO₂NHBu^{t}, 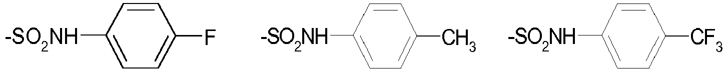
- Urea (-NHCONH₂) which may be optionally substituted
- Thiourea (-NHCSNH₂) which may be optionally substituted
- Sulfonylurea (-NHSO₂NH₂) which may be optionally substituted
- Oxo (=O) when oxygen is bonded through double bond to a carbon atom
- Oxyimino (=N-O-A) where the nitrogen is bonded through double bond to a carbon atom which is attached to the rest of the molecule and A can be hydrogen, or optionally substituted straight or branched lower alkyl, cycloalkyl, aryl, heteroaryl and heterocyclyl
- Hydroxamic acid (-CONHOH)
- Acyl (-COCH₃)
- Trifluoromethyl carbonyl (-COCF₃)
- Cyano (-CN)
- Amidino -C(=NH)NH₂ which may be optionally substituted
- Guanidino -NHC(=NH)NH₂ which may be optionally substituted
- Aryloxy
- Heterocyclyl
- Heteroaryl
- Heterocyclyloxy
- Heteroaryloxy
- Heterocyclylalkyloxy
- Trialkylammonium

The substituent mentioned above could be substituted at the carbon atom or at the free N-atom of the molecule as appropriate.

A group of preferred examples of compounds of the formula (I), when Y = OR¹, are from the following Table 1. 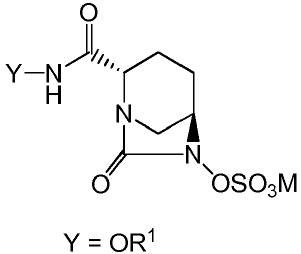 * = point of attachment with O

**Table 1: List of compounds**

| **Compound No.** | **M** | **R¹** |
|---|---|---|
| **26** | **H** | 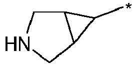 |
| **27** | **H** | 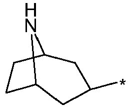 |
| **31** | **H** | 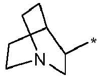 |
| **32** | **H** | 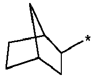 |
| **33** | **H** | 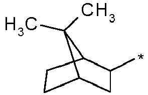 |
| **34** | **H** | 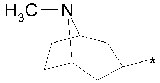 |
| **42** | **H** | 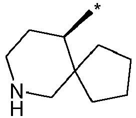 |
| **43** | **H** | 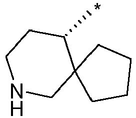 |
| **44** | **H** | 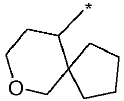 |
| **60** | **H** | 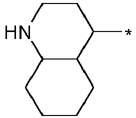 |
| **62** | **H** | 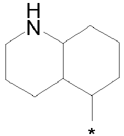 |
| **63** | **H** | 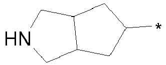 |

A group of preferred examples of compounds of the formula (I), when Y = NR²R³, are from the following Table 2. 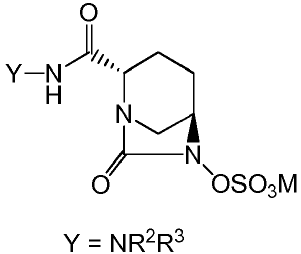 * = point of attachment with N

**Table 2: List of compounds**

| **Compound No.** | **M** | **R²** | **R³** |
|---|---|---|---|
| **90** | **H** | **H** | 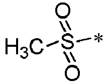 |
| **92** | **H** | **H** | 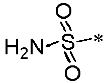 |

It is also an object of this invention to provide a combination of a compound of general formula (I) having antibacterial activity with another existing antibacterial agent, thus causing synergistic effect and the use of the same as drugs for the treatment of bacterial infections.

It is another object of the invention to provide methods for preparing the compounds of the invention of formula (I).

It is a further object of the invention to provide pharmaceutical compositions comprising a compound of formula (I) of this invention (some of which directly inhibit β-lactamase enzymatic function and others of which do not directly inhibit β-lactamase's enzymatic function (at pharmaceutically accessible concentrations) as an active ingredient in combination with an antibiotic (e.g., a β-lactam antibiotic or some other non β-lactam antibiotic) and a suitable amount of pharmaceutically acceptable carrier or diluent, so as to provide a form for proper administration to a patient. These compositions can be administered by parenteral, in particular intramuscular route, oral, sublingual, rectal, aerosol or by local route in a topical application on the skin and the mucous membranes. Suitable pharmaceutical vehicles include excipients such as starch, glucose, lactose, sucrose, gelatin, gum arabic, malt, rice, flour, chalk, silica gel, sodium stearate, glycerol monostearate, talc, sodium chloride, dried skim milk, glycerol, propylene glycol, water, and ethanol. Other examples of suitable pharmaceutical vehicles have been described in the art (Remington's Science and Practice of Pharmacy, 21st Edition, 2006). Compositions of the present disclosure, if desired, can also contain minor amounts of wetting, dispersing or emulsifying agents, or pH buffering agents, and preservatives. In addition, auxiliary, stabilizing, thickening, lubricating, and coloring agents can be included. Pharmaceutical compositions can be formulated in a conventional manner. Proper formulation is dependent upon the route of administration chosen. The present pharmaceutical compositions can take the form of injectable preparations, suspensions, emulsions, sugar-coated tablets, pellets, gelatin-capsules, capsules containing liquids, powders, granules, sustained-release formulations, suppositories, aerosols, sprays, ointments, creams or any other form suitable for use.

The parenteral administration which includes intramuscular, intraperitonial, subcutaneous and intravenous use, sterile solutions of the active ingredient are usually prepared and the pH of the solutions are suitably adjusted and buffered. For intravenous use, the total concentration of solutes should be controlled to render the preparation isotonic. Suitable solvents include saline solution (e.g., 0.9% NaCl solution) and apyrogenic sterile water. Pharmaceutical compositions for oral delivery can be, for example, in the form of tablets, lozenges, aqueous or oily suspensions, granules, powders, emulsions, capsules, syrups, or elixirs. Orally administered compositions can contain one or more optional agents, for example, sweetening agents such as fructose, aspartame, or saccharin, flavoring agents such as peppermint, oil of wintergreen, cherry, coloring agents, and preserving agents to provide a pharmaceutically palatable preparation. Moreover, when in tablet form, the compositions can be coated to delay disintegration and absorption in the gastrointestinal tract, thereby providing a sustained action over an extended period of time. Oral compositions can include standard vehicles such as mannitol, lactose, starch, magnesium stearate, sodium saccharin, cellulose, magnesium and carbonate. For oral liquid preparations, for example, suspensions, elixirs, and solutions, suitable carriers, excipients, or diluents include water, saline, alkyleneglycols (e.g. propylene glycol), polyalkylene glycols (e.g., polyethylene glycol), oils, alcohols, slightly acidic buffers ranging from about pH 4 to about pH 6 (e.g., acetate, citrate, ascorbate ranging from about 5 mM to about 50 mM). Additionally, flavoring agents, preservatives, coloring agents, bile salts, and acylcarnitines can be added.

For topical formulations of compounds of the present invention, creams, gels, ointments or viscous lotions can be used as appropriate delivery forms. Topical delivery systems also include transdermal patches containing at least one compound of formula (I) to be administered. Delivery through the skin can be achieved by diffusion or by more active energy sources such as iontophoresis or electrotransport. Formulations of a compound of the present invention, for topical use, such as in creams, ointments, and gels, can include an oleaginous or water soluble ointment base, for example, topical compositions can include vegetable oils, animal fats, and in certain embodiments, semisolid hydrocarbons obtained from petroleum. Topical compositions can further include white ointment, yellow ointment, cetyl esters wax, oleic acid, olive oil, paraffin, petrolatum, white petrolatum, spermaceti, starch glycerite, white wax, yellow wax, lanolin, and glyceryl monostearate. Various water-soluble ointment bases can also be used, including glycol ethers and derivatives, polyethylene glycols, polyoxyl 40 stearate, and polysorbates.

In a pharmaceutical composition containing a compound of this invention, the weight ratio of active ingredient to carrier will normally be in the range of 1:20 to 20:1. The administered daily dose varies according to the illness treated, and the administration route. However in most instances, an effective dose (e.g., in some instances, a β-lactamase inhibiting dose) of a compound of formula (I) or a pharmaceutically acceptable salt thereof will be a daily dose in the range from about 1 to about 500 mg per kilogram of body weight orally, and from about 1 to about 500 mg per kilogram of body weight parenterally. The weight ratio of the compound of present invention and an antibiotic (if it is being administered with an antibiotic, e.g., a β-lactam antibiotic or some other non β-lactam antibiotic) will normally be in the range from 1:20 to 20:1.

In some aspects of the present invention, an additional object is to provide at least one compound chosen from formula (I) or a pharmaceutically acceptable salt thereof in combination with a known β-lactam antibiotic for use in a method for the treatment of bacterial infections caused by β-lactamase producing bacteria in a patient in need of such treatment comprising administering to the patient a therapeutically effective amount of said at least one compound chosen from formula (I) or a pharmaceutically acceptable salt thereof in combination with a known β-lactam antibiotic. In such an aspect of the present invention, the compounds increase the antibacterial effectiveness of β-lactamase susceptible β-lactam antibiotics, that is, they increase the effectiveness of the antibiotic against infections caused by β-lactamase producing microorganisms in mammalian subjects, particularly in human. In these aspects of the present invention, this makes the compounds of formula (I) and pharmaceutically acceptable salts thereof, valuable for co-administration with β-lactam antibiotics. In the treatment of a bacterial infection in such aspects of the present invention, said compounds of formula (I) or a pharmaceutically salt thereof can be mixed with the β-lactam antibiotic, and the two agents thereby administered simultaneously. When co-administered with a β-lactam antibiotic in such aspects of the present invention, the combination of the compound of the invention and the antibiotic can provide a synergistic effect. The term 'synergystic effect' refers to the effect produced when two or more agents are co-administered is greater than the effect produced when the agents are administered individually. Alternatively, the compound of formula (I) or a salt thereof can be administered as a separate agent during a course of treatment with the antibiotic.

Therapeutically effective amount' refers to the amount of a compound that, when administered to a subject for treating a disease, or at least one of the clinical symptoms of a disease, is sufficient to affect such treatment of the disease, disorder, or symptom. The therapeutically effective amount can vary depending, for example, on the compound, the disease, disorder, and/or symptoms of the disease, severity of the disease, disorder, and/or symptoms of the disease, the age, weight, and/or health of the patient to be treated, and the judgement of the prescribing physician.

The term 'β-lactam antibiotic' refers to a compound with antibiotic property that contains a β-lactam functionality. Examples of β-lactam antibiotics which can be used in combination with the compounds of the present invention represented by formula (I) are commonly marketed penicillins, cephalosporins, penems, carbapenems and monobactams.

Examples of β-lactam antibiotics which can be used in combination with the compounds of the present invention represented by formula (I) are commonly used penicillins, such as amoxicillin, ampicillin, azlocillin, mezlocillin, apalcillin, hetacillin, bacampicillin, carbenicillin, sulbenicillin, ticarcillin, piperacillin, methicillin, ciclacillin, talampicillin, oxacillin, cloxacillin, dicloxacillin and commonly used cephalosporins such as cephalothin, cephaloridine, cefaclor, cefadroxil, cefamandole, cefazolin, cephalexin, cephradine, cephapirin, cefuroxime, cefoxitin, cephacetrile, cefotiam, cefotaxime, cefatriazine, cefsulodin, cefoperazone, ceftizoxime, cefmenoxime, cefmetazole, cephaloglycin, cefonicid, cefodizime, cefpirome, cefepime, ceftazidime, cefpiramide, ceftriaxone, cefbuperazone, cefprozil, cefixime, ceftobiprole, ceftaroline, cefalonium, cefminox, ceforanide, cefuzonam, cefoxitin, cefotetan, loracarbef, cefdinir, cefditoren, cefetamet, cefcapene, cefdaloxime, ceftibuten, cefroxadine, latamoxef (moxalactam), and CXA-101. From the carbapenem class of β-lactam antibiotics such as imipenem, meropenem, panipenem, biapenem, doripenem, ertapenem could be used. From monobactam class of β-lactam antibiotics such as aztreonam, carumonam, and tigemonam could be used as the combination partner of antibiotic. Examples of antibiotics (which are not β-lactam antibiotics) which can be used in combination with the compounds of the present invention (i.e., compounds of formula (I) above, salts thereof, and solvates of such compounds and salts) include aminoglycosides, quinolones, tetracyclines, glycylcyclines, glycopeptides, lipopeptides, macrolides, ketolides, lincosamides, streptogramin, oxazolidinones, polymyxins, and other compounds known to have antibacterial properties.

'Pharmaceutically acceptable solvate' refers to a molecular complex of a compound with one or more solvent molecules in a stoichiometric or non-stoichiometric amount. Such solvent molecules are those commonly used in the pharmaceutical art, which are known to be innocuous to recipient, e.g., water, and ethanol. A molecular complex of a compound or moiety of a compound and a solvent can be stabilized by non-covalent intra-molecular forces such as, for example, electrostatic forces, Van der Waals forces or hydrogen bonds. The term hydrate refers to a complex where the one or more solvent molecules are water.

Among the compounds of formula (I), a particular subject of the invention is the compounds with the following names. The following examples illustrate the invention, and are not intended to be limiting of its scope.

The non-limiting examples of the compounds of the present invention are:
(*2S*,*5R*)-*N*-(3-azabicyclo[3.1.0]hex-6-yloxy)-7-oxo-6-(sulfooxy)-1,6-diazabicyclo[3.2.1]octane-2-carboxamide
(2*S*,5*R*)-*N*-(decahydroquinolin-5-yloxy)-7-oxo-6-(sulfooxy)-1,6-diazabicyclo[3.2.1]octane-2-carboxamide
(2*S*,5*R*)-*N*-(octahydrocyclopenta[*c*]pyrrol-5-yloxy)-7-oxo-6-(sulfooxy)-1,6-diazabicyclo[3.2.1]octane-2-carboxamide
(2*S*,5*R*)-*N*-(bicyclo[2.2.1]hept-2-yloxy)-7-oxo-6-(sulfooxy)-1,6-diazabicyclo[3.2.1]octane-2-carboxamide
(2*S*,5*R*)-*N*-[(7,7-dimethylbicyclo[2.2.1]hept-2-yl)oxy]-7-oxo-6-(sulfooxy)-1,6-diazabicyclo[3.2.1]octane-2-carboxamide
(2*S*,5*R*)-*N*-(1-azabicyclo[2.2.2]oct-3-yloxy)-7-oxo-6-(sulfooxy)-1,6-diazabicyclo[3.2.1]octane-2-carboxamide
(2*S*,5*R*)-*N*-(8-azabicyclo[3.2.1]oct-3-yloxy)-7-oxo-6-(sulfooxy)-1,6-diazabicyclo[3.2.1]octane-2-carboxamide
(2*S*,5*R*)-*N*-[(8-methyl-8-azabicyclo[3.2.1]oct-3-yl)oxy]-7-oxo-6-(sulfooxy)-1,6-diazabicyclo[3.2.1]octane-2-carboxamide
(2*S*,5*R*)-*N*-(7-azaspiro[4.5]dec-10-yloxy)-7-oxo-6-(sulfooxy)-1,6-diazabicyclo[3.2.1]octane-2-carboxamide
(2*S*,5*R*)-*N*-(7-oxaspiro[4.5]dec-10-yloxy)-7-oxo-6-(sulfooxy)-1,6-diazabicyclo[3.2.1]octane-2-carboxamide
(2*S*,5*R*)-*N*'-(methylsulfonyl)-7-oxo-6-(sulfooxy)-1,6-diazabicyclo[3.2.1]octane-2-carbohydrazide
2-{[(2*S*,5*R*)-7-oxo-6-(sulfooxy)-1,6-diazabicyclo[3.2.1]oct-2-yl]carbonyl}hydrazinesulfonamide

The compounds of the present invention of formula (I, when Y = OR¹) can be readily prepared by the following reaction Scheme 2 and examples using readily available starting materials, reagents and conventional synthesis procedures known to those of ordinary skill in this art. The methods differ according to the kind of substituted hydroxylamines of general formula (V) used to prepare the bicyclic diazaoctane derivatives. The bicyclic intermediate acid (VI) may be prepared following the patent literature WO 2009/091856.

Compounds of general formula (I, Y = OR¹, M = H) can be prepared by coupling an appropriately substituted hydroxylamine (V) with the bicyclic acid (VI) in presence of a suitable coupling reagent to give the desired intermediate (VII). The coupling reagents useful for carrying out this step include, but are not limited to, EDCI, HOBT-DCC, HATU, HOBT, and PyBop. The organic solvents useful in the reaction are not particularly limited and include any of those which do not adversely affect the coupling reaction. Typical solvents include DCM, chloroform, dimethylformamide, dimethylacetamide, tetrahydrofuran, acetonitrile, dimethylsulfoxide, and acetonitrile. The reaction is normally carried out at a temperature of from about 0 °C to about 30 °C and preferably at room temperature under nitrogen. After completion of the reaction the desired product can be easily separated by conventional methods such as column chromatography, crystallization or similar methods.

In the following step, the intermediate (VII) could be converted to compound (VIII) under an atmosphere of hydrogen or hydrogen mixed with an inert diluent such as nitrogen or argon in the presence of a hydrogenation catalyst. The catalysts used in this hydrogenation reaction are the type of agents known in the art for this kind of deprotection and typical examples are the noble metals, such as nickel, palladium, platinum and rhodium. Examples of the catalysts are platinum, platinum oxide, palladium, palladium oxide. The catalyst is usually present in the amount from about 1 to about 50 weight percent and preferably from about 5 to about 10 weight percent based on the compound of formula (I). It is often convenient to suspend the catalyst on an inert support. A particularly convenient catalyst is palladium suspended on an inert support such as carbon, e.g 10 % by weight palladium on carbon. This reaction may be conveniently effected at ambient temperature at 40 psi until reaction is complete (2 to 12 hours). Suitable solvents for this reaction are those which substantially dissolve the starting material of the formula (VII), are sufficiently volatile to be removed by evaporation and do not themselves suffer hydrogenation. Examples of such solvents include methanol, ethanol, dioxane, ethyl acetate, tetrahydrofuran or a mixture of these solvents. Upon completion, the hydroxy intermediate (VIII) can be purified by silica gel column chromatography or in many cases can be directly carried out to the next step without further purification.

Sulfation of the intermediate (VIII) can be achieved using a sulfating reagent (e.g., pyridine-SO₃ complex, ClSO₃H and DMF-SO₃ complex) in an appropriate solvent (e.g., pyridine or 2-picoline), e.g., as described in the literature (US 4337197 A1, J. Am. Chem. Soc., 1982, 104, 6053-6060). Thus, SO₃-Py complex can be added to a solution of the intermediate (VIII) in a solvent in excess amount, if desired, to force the reaction to completion. The organic solvents useful for this transformation are not particularly limited and include those which do not adversely affect the reaction. Typical solvents include, but not limited to, pyridine, dimethyl formamide, dimethylacetamide, acetonitrile, and DCM. The transformation can be carried out at from 10 °C to 40 °C, and more preferably at room temperature. The product (IX) can be isolated by standard procedure that is by filtering the reaction mixture, concentrating the filtrate, suspending the concentrate in a saturated aqueous potassium dihydrogenphosphate solution, washing the aqueous layer with ethyl acetate, adding excess amount of tetrabutylammonium hydrogen sulfate to the aqueous layer, extracting the mixture with organic solvent, such as ethyl acetate, combining the organic layers, drying and concentrating to provide the tetrabutylammonium salt intermediate. Treating the intermediate (IX) with an acid to obtain a compound of formula (Ia, M = H), wherein R¹ has the same definition as in formula (I). Suitable organic acids include trifluoroacetic acid, methanesulfonic acid, trifluoromethane sulfonic acid, and formic acid. The treatment is suitably conducted at a temperature in a range from about -10 °C to about 30 °C and is typically conducted at a temperature in a range of from about 0 °C to about 10 °C.

The substituted hydroxylamines (V) used in the invention can be prepared by a two steps procedure using the methods well known in the art. Thus, the alcohol (II) is reacted with *N-*hydroxyphthalimide (III) in presence of PPh₃ under Mitsunobu conditions to provide the intermediate (IV). Treating (IV) with hydrazine hydrate in presence of a solvent provides the desired substituted hydroxylamine (V) which can be used without further purification (Scheme 1).

Similarly, compounds of general formula (I, Y = NR²R³, M = H) can be prepared by coupling an appropriately substituted hydrazine (Va) with the bicyclic acid (VI) in presence of a suitable coupling reagent to give the desired intermediate (VIIa). Utilizing the intermediate (VIIa) and carrying out similar sets of experiments as described for (Ia), the desired compounds (Ib, M = H) of the present invention can be obtained as shown in Scheme 3. The substituted hydrazines (Va) used in the present invention can be obtained from the commercial source or can be prepared by the known literature procedure.

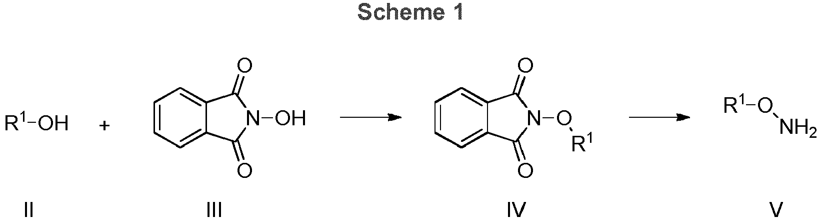

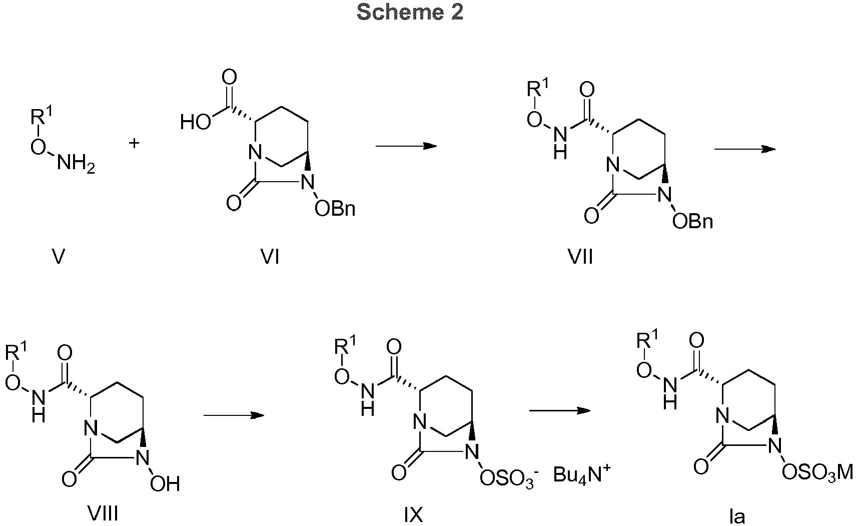

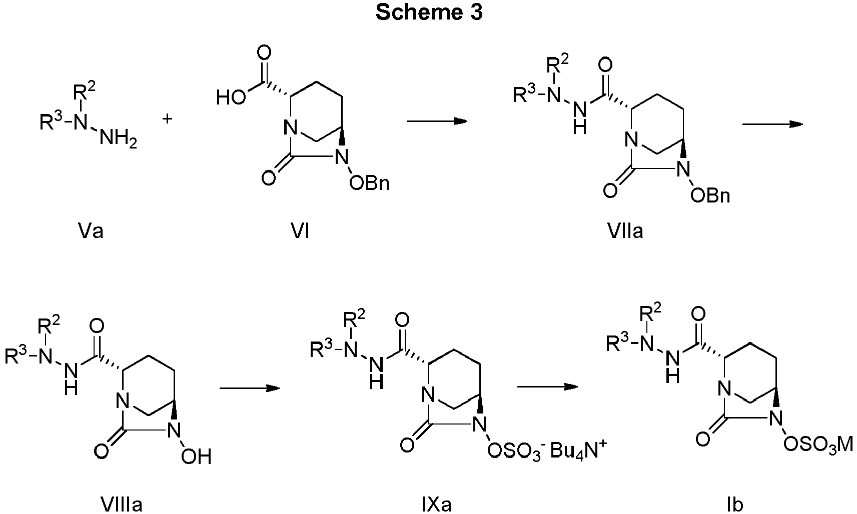

### Examples

The compounds of Examples 1-3, 5 and 7-9 do not form part of the invention.

In the examples, the following abbreviations have been used:
Bn: benzyl
Boc: *N*-*tert*-butoxycarbonyl
br s: broad singlet
CDCl₃: deuterated chloroform
CD₃OD: deuterated methanol
d: doublet
D₂O: deuterium oxide
DCC: *N*,*N*'-dicyclohexylcarbodiimide
DCM: dichloromethane
DIAD: diisopropyl azodicarboxylate
DMAP: 4-dimethylaminopyridine
EDCI: 1-(3-dimethylamino-propyl)-3-ethylcarbodiimide hydrochloride
EI: electron impact
ES: electron spray
FAB: fast atom bombardment
g: gram(s)
h: hour(s)
HOBT: *N*-hydroxybenzotriazole
HATU: 2-(7-aza-1*H*-benzotriazol-1-yl)-1,1,3,3-tetramethyluronium hexafluorophosphate
HPLC: high-performance liquid chromatography
Hz: Hertz
J: coupling constant
m: multiplet
mL: milliliter(s)
mmol: millimole(s)
MHz: megahertz
MS: mass spectrometry
*m*/*z*: mass-to-charge ratio
NMR: nuclear magnetic resonance
Pd/C: palladium on carbon
PyBop: (benzotriazol-1-yloxy)tripyrrolidinophosphonium hexafluorophosphate s: singlet
t: triplet
TFA: trifluoroacetic acid
THF: tertrahydrofuran
δ: chemical shift in parts per million (ppm) by frequency

### Example 1

### (2S,5R)-7-Oxo-N-[(3S)-pyrrolidin-3-yloxy]-6-(sulfooxy)-1,6-diazabicyclo[3.2.1]octane-2-carboxamide

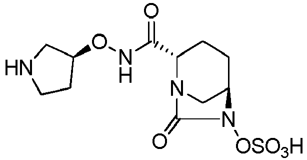

### Step 1. tert-Butyl (3S)-3-[({[(2S,5R)-6-(benzyloxy)-7-oxo-1,6-diazabicyclo[3.2.1]oct-2-yl]carbonyl}amino)oxy]pyrrolidine-1-carboxylate (8)

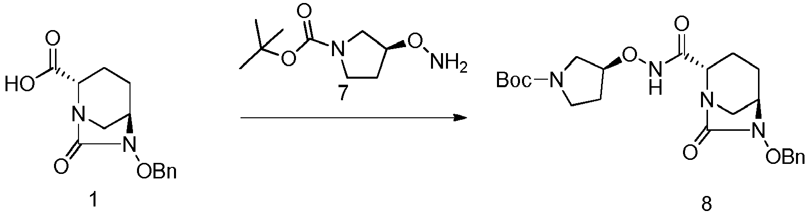

To a mixture of (2*S*,5*R*)-6-(benzyloxy)-7-oxo-1,6-diazabicyclo[3.2.1]octane-2-carboxylic acid **1** (0.150 g, 0.543 mmol, US 2005/20572 A1) in DCM (4.0 mL) were added *tert-*butyl (3*S*)-3-(aminooxy)pyrrolidine-1-carboxylate **7** (0.164 g, 0.814 mmol, WO 2008/67481 A1), 1-hydroxybenzotriazole (0.110 g, 0.814 mmol) and 1-ethyl-(3-dimethylaminopropyl) carbodiimide hydrochloride (0.156 g, 0.814 mmol) sequentially at room temperature. The mixture was stirred at room temperature overnight, diluted with DCM and concentrated to provide a residue which was subjected to chromatography to give **8** (0.22 g, 88 %) as a white foam.
¹H NMR (400 MHz, CDCl₃): δ 1.46 (9H, s), 1.61 (1H, m), 1.93 (3H, m), 2.17 (1H, m), 2.30 (1H, m), 2.72 (1H, d, *J* = 11.6 Hz), 2.99 (1H, m), 3.45 (5H, m), 3.99 (1H, m), 4.60 (1H, m), 4.92 (1H, d, *J* = 11.6 Hz), 5.04 (1H, d, *J* = 11.6 Hz), 7.42 (5H, m), 9.00 (1H, br s).
MS (ES⁻) *m*/*z*: [M-H]⁻ calcd for C₂₃H₃₁N₄O₆: 459.22. Found: 459.08.

### Step 2. tert-Butyl (3S)-3-[({[(2S,5R)-6-hydroxy-7-oxo-1,6-diazabicyclo[3.2.1]oct-2-yl]carbonyl}amino)oxy]pyrrolidine-1-carboxylate (9)

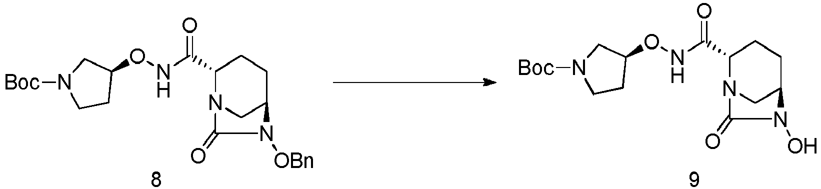

A mixture of *tert-*butyl (3*S*)-3-[({[(2*S*,5*R*)-6-(benzyloxy)-7-oxo-1,6-diazabicyclo[3.2.1] oct-2-yl]carbonyl}amino)oxy]pyrrolidine-1-carboxylate **8** (0.22 g, 0.48 mmol) and Pd/C (0.070 g) in methanol (10 mL) was hydrogenated at 1 atm at room temperature for 3 h. The mixture was filtered through Celite pad and concentrated to provide **9** (0.19 g, quant. yield) as a light yellow foam.
¹H NMR (400 MHz, CD₃OD): δ 1.46 (9H, m), 1.75-2.20 (6H, m), 3.03 (1H, d, *J* = 11.6 Hz), 3.17 (1H, m), 3.44 (3H, m), 3.63 (1H, d, *J =* 13.2 Hz), 3.69 (1H, m), 3.86 (1H, d, *J =* 7.2 Hz), 4.58 (1H, t, *J* = 3.6 Hz). 2 protons were not observed in CD₃OD.
MS (ES⁻): *m*/*z* [M-H]⁻ calcd for C₁₆H₂₅N₄O₆: 369.18. Found: 369.06.

### Step 3. tert-Butyl (3S)-3-[({[(2S,5R)-7-oxo-6-(sulfooxy)-1,6-diazabicyclo [3.2.1] oct-2-yl]carbonyl}amino)oxy]pyrrolidine-1-carboxylate (10)

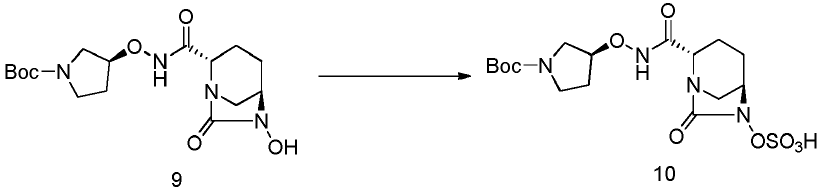

To a mixture of *tert-*butyl (3*S*)-3-[({[(2*S*,5*R*)-6-hydroxy-7-oxo-1,6-diazabicyclo[3.2.1]oct-2-yl]carbonyl}amino)oxy]pyrrolidine-1-carboxylate **9** (0.19 g, 0.51 mmol) in pyridine (7.0 mL) was added sulfur trioxide pyridine complex (0.326 g, 2.05 mmol). The mixture was stirred at room temperature for 23 h and concentrated to provide a residue which was subjected to chromatography to give **10** (0.11 g, 48 %) as a white solid.
¹H NMR (400 MHz, CD₃OD): δ 1.47 (9H, s), 1.80-2.20 (6H, m), 3.07 (1H, d, *J =* 12 Hz), 3.27 (1H, m), 3.44 (3H, m), 3.60 (1H, m), 3.92 (1H, d, *J =* 11.6 Hz), 4.14 (1H, m), 4.59 (1H, m). 2 protons were not observed in CD₃OD.
MS (ES⁻): *m*/*z* [M-H]⁻ calcd for C₁₆H₂₅N₄O₉S: 449.13. Found: 448.99.

### Step 4. (2S,5R)-7-Oxo-N-[(3S)-pyrrolidin-3-yloxy]-6-(sulfooxy)-1,6-diazabicyclo[3.2.1 ]octane-2-carboxamide

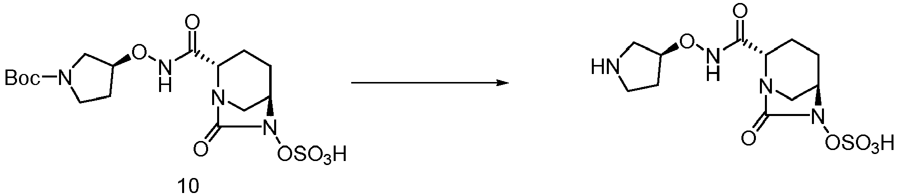

To a mixture of *tert-*butyl (3*S*)-3-[({[(2*S*,5*R*)-7-oxo-6-(sulfooxy)-1,6-diazabicyclo[3.2.1]oct-2-yl]carbonyl}amino)oxy]pyrrolidine-1-carboxylate **10** (0.11 g, 0.24 mmol) in DCM (4.0 mL) was added trifluoroacetic acid (0.20 mL) at 0°C. The mixture was stirred at 0°C for 1 h, concentrated and washed with ether. The white solid was collected by centrifugation. The crude product was purified by preparative HPLC to provide the title compound (30.4 mg, 36 %) as a white solid.
¹H NMR (400 MHz, D₂O): δ 1.74-1.83 (2H, m), 1.91-2.11 (3H, m), 2.18-2.22 (1H, m), 2.98 (1H, d, *J =* 12 Hz), 3.17 (1H, m), 3.27-3.34 (3H, m), 3.45 (1H, dd, *J =* 0.8 Hz, 13.6 Hz), 3.94 (1H, m), 4.06 (1H, m), 4.71 (1H, m). 3 protons were not observed in D₂O.
HPLC: 96.77 %
MS (ES⁻): *m*/*z* [M-H]⁻ calcd for C₁₁H₁₇N₄O₇S: 349.08. Found: 348.95.

### Example 2

### (2S,5R)-7-Oxo-N-[(3S)-piperidin-3-yloxy] -6-(sulfooxy)-1,6-diazabicyclo[3.2.1]octane-2-carboxamide

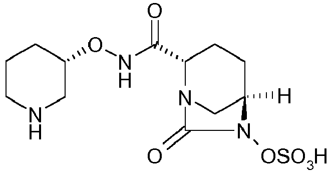

### Step 1. tert-Butyl (3S)-3-[({[(2S,5R)-6-(benzyloxy)-7-oxo-1,6-diazabicyclo[3.2.1]oct-2-yl]carbonyl}amino)oxy]piperidine-1-carboxylate (17)

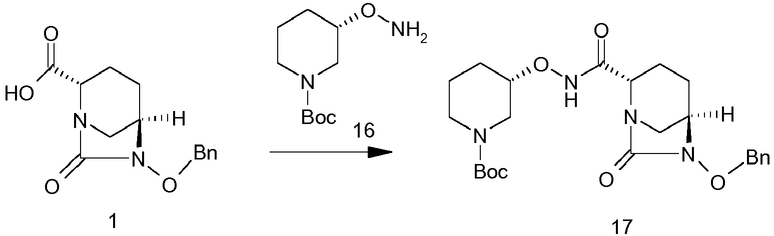

To a solution of (2*S*,5*R*)-6-(benzyloxy)-7-oxo-1,6-diazabicyclo[3.2.1]octane-2-carboxylic acid **1** (0.20 g, 0.72 mmol) in dry DCM (20 mL) were added *tert-*butyl (3*S*)-3-(aminooxy)piperidine-1-carboxylate **16** (0.19 g, 0.86 mmol, J. Med. Chem. 2008, 51, 4601-4608), 1-hydroxybenzotriazole (0.14 g, 1.03 mmol) and 1-ethyl-(3-dimethylaminopropyl)carbodiimide hydrochloride (0.20 g, 1.03 mmol) at room temperature. The reaction mixture was stirred at room temperature overnight and concentrated under vacuum. The residue was purified by column chromatography to give *tert-*butyl (3*S*)-3-[({[(2*S*,5*R*)-6-(benzyloxy)-7-oxo-1,6-diazabicyclo[3.2.1]oct-2-yl]carbonyl}amino)oxy]piperidine-1-carboxylate **17** (0.28 g, 82 %) as a white solid.
¹H NMR (400 MHz, CDCl₃): δ 1.46 (9H, s), 1.61 (1H, m), 1.83 (2H, m), 2.01 (4H, m), 2.31 (1H, m), 2.79 (1H, d, J = 11.2 Hz), 2.99 (3H, m), 3.30 (1H, s), 3.60-4.11 (4H, m), 4.88 (1H, d, J = 11.6 Hz), 5.05 (1H, d, J = 11.6 Hz), 7.39 (5H, m), 9.96 (1H, br s).

### Step 2. tert-Butyl (3S)-3-[({[(2S,5R)-6-hydroxy-7-oxo-1,6-diazabicyclo[3.2.1]oct-2-yl]carbonyl}amino)oxy]piperidine-1-carboxylate (18)

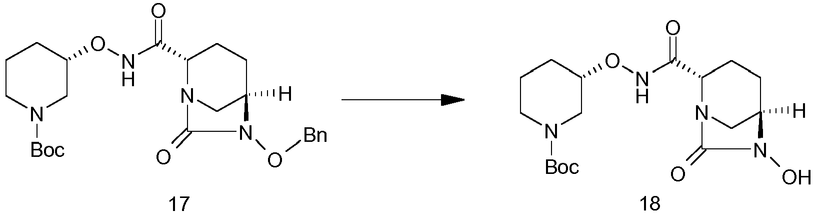

To a solution of *tert-butyl* (3*S*)-3-[({[(2*S*,5*R*)-6-(benzyloxy)-7-oxo-1,6-diazabicyclo[3.2.1]oct-2-yl]carbonyl}amino)oxy]piperidine-1-carboxylate **17** (0. 28 g, 0.59 mml) in methanol (20 mL) was added 5 % Pd/C (0.25 g). The mixture was hydrogenated at 35 psi hydrogen atmosphere at room temperature for 1 h. The catalyst was filtered out through Celite, and the filtrate was evaporated to give *tert-butyl* (3*S*)-3-[({[(2*S*,5*R*)-6-hydroxy-7-oxo-1,6-diazabicyclo[3.2.1]oct-2-yl]carbonyl}amino)oxy]piperidine-1-carboxylate **18** (0.22 g, 97 %) as a white solid.
¹H NMR (400 MHz, CDCl₃): δ 1.45 (9H, s), 1.68-1.98 (6H, m), 2.05 (1H, m), 2.22 (1H, m), 3.03 (1H, d, J = 12.0 Hz), 3.13 (1H, d, J = 11.6 Hz), 3.28-3.59 (4H, m), 3.71 (1H, s), 3.87 (2H, m), 2 protons were not observed in CD₃OD.

### Step 3. tert-Butyl (3S)-3-[({[(2S,5R)-7-oxo-6-(sulfooxy)-1,6-diazabicyclo[3.2.1]oct-2-yl]carbonyl}amino)oxy]piperidine-1-carboxylate pyridine salt (19)

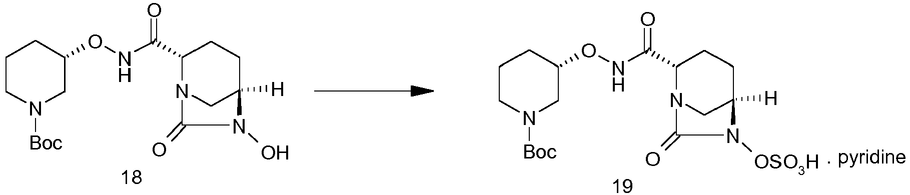

To a solution of *tert-*butyl (3*S*)-3-[({[(2*S*,5*R*)-6-hydroxy-7-oxo-1,6-diazabicyclo[3.2.1]oct-2-yl]carbonyl}amino)oxy]piperidine-1-carboxylate **18** (0.22 g, 0.57 mmol) in dry pyridine (8 mL) under nitrogen atmosphere was added sulfur trioxide pyridine complex (0.40 g, 2.51 mmol). The mixture was stirred at room temperature for 20 h, filtered and evaporated to give *tert-*butyl (3*S*)-3-[({[(2*S*,5*R*)-7-oxo-6-(sulfooxy)-1,6-diazabicyclo[3.2.1]oct-2-yl]carbonyl}amino)oxy]piperidine-1-carboxylate pyridine salt **19** (0.23 g crude) which was used in the next step without purification.

### Step 4. N,N,N-Tributylbutan-1-aminium ({[(2S,5R)-2-({[(3S)-1-(tert-butoxycarbonyl)piperidin-3-yl]oxy}carbamoyl)-7-oxo-1,6-diazabicyclo[3.2.1]oct-6-yl]oxy}sulfonyl)oxidanide (20)

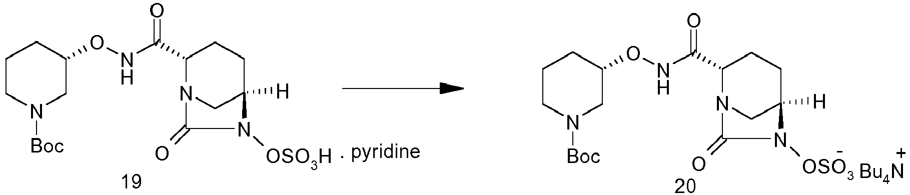

*tert*-Butyl (3*S*)-3-[({[(2*S*,5*R*)-7-oxo-6-(sulfooxy)-1,6-diazabicyclo[3.2.1]oct-2-yl]carbonyl}amino)oxy]piperidine-1-carboxylate pyridine salt **19** (0.23 g, 0.42 mmol) was introduced into a concentrated aqueous solution of monosodium dihydrogen phosphate solution (8 mL) so as to obtain a pH of 4. The mixture was washed with ethyl acetate, then added tetrabutyl ammonium hydrogen sulfate (0.088 g, 0.26 mmol) and stirred at room temperature for 10 min. The mixture was extracted with ethyl acetate (3 x 20 mL), and the extracts were combined, dried over sodium sulfate and evaporated to give *N*,*N,N*-tributylbutan-1-aminium ({[(2*S*,5*R*)-2-({[(3*S*)-1-(*tert*-butoxycarbonyl)piperidin-3-yl]oxy}carbamoyl)-7-oxo-1,6-diazabicyclo[3.2.1]oct-6-yl]oxy}sulfonyl)oxidanide **20** (0.23 g, 52.5 %) as a white solid.
¹H NMR (400 MHz, CDCl₃): δ 0.98 (12H, t, J = 7.2 Hz), 1.42 (17H, m), 1.65 (8H, m), 1.77 (4H, m), 2.05 (3H, m), 2.33 (1H, m), 2.85 (1H, d, J = 11.6 Hz), 2.96 (2H, m), 3.24 (9H, m), 3.65 (1H, m), 3.95 (2H, m), 4.10 (1H, m), 4.13 (1H, s), 10.00 (1H, br s).

### Step 5. (2S,5R)-7-Oxo-N-[(3S)-piperidin-3-yloxy]-6-(sulfooxy)-1,6-diazabicyclo[3.2.1 ]octane-2-carboxamide

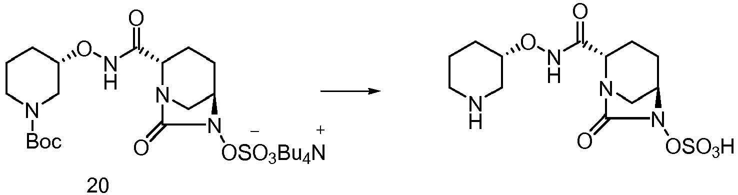

To a solution of *N*,*N*,*N*-tributylbutan-1-aminium ({[(2S,5*R*)-2-({[(3*S*)-1-(*tert-*butoxycarbonyl)piperidin-3-yl]oxy}carbamoyl)-7-oxo-1,6-diazabicyclo[3.2.1]oct-6-yl]oxy}sulfonyl)oxidanide **20** (0.23 g, 0.32 mmol) in DCM (15 mL) was added trifluoroacetic acid (0.64 mL, 8.32 mmol) dropwise at 0 °C. The reaction mixture was stirred for 1 h, then evaporated. Ether was added to the residue and the resulting white precipitate was collected by centrifugation. The solid was triturated with acetonitrile (2 x) and the white solid was collected by centrifugation. The white solid was purified by HPLC and freeze-dried to give (2*S*,*5R*)-7**-**oxo-*N*-[(3*S*)-piperidin-3-yloxy]-6-(sulfooxy)-1,6-diazabicyclo[3.2.1]octane-2-carboxamide (0.008 g, 6.8 %) as a white solid.
¹H NMR (400 MHz, CD₃OD): δ 1.60-1.78 (3H, m), 1.80-2.08 (5H, m), 2.92-3.04 (2H, m), 3.14-3.26 (2H, m), 3.30 (1H, d, J = 13.2 Hz), 3.94-4.02 (2H, m), 4.08 (1H, d, s), 4.18 (1H, s), 3 protons were not observed in CD₃OD.
HPLC: 97.05 %
MS (ES⁻): m/z [M]⁻= 363.02

### Example 3

### (2S,5R)-N-(2-Aminoethoxy)-7-oxo-6-(sulfooxy)-1,6-diazabicyclo[3.2.1]octane-2-carboxamide

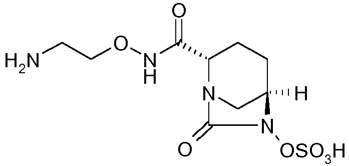

### Step 1. tert-Butyl {2-[({[(2S,5R)-6-(benzyloxy)-7-oxo-1,6-diazabicyclo[3.2.1]oct-2-yl]carbonyl}amino)oxy]ethyl}carbamate (42)

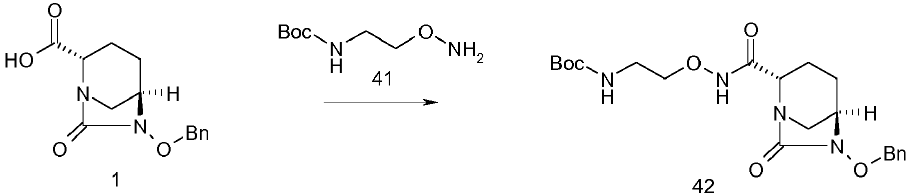

To a mixture of (2*S*,5*R*)-6-(benzyloxy)-7-oxo-1,6-diazabicyclo[3.2.1]octane-2-carboxylic acid 1 (0.150 g, 0.543 mmol, US 2005/20572 A1) in DCM (4.0 mL) was added *tert-*butyl [2-(aminooxy)ethyl]carbamate **41** (0.143 g, 0.814 mmol, US 2005/54701 A1), 1-hydroxybenzotriazole (0.110 g, 0.814 mmol), 1-ethyl-(3-dimethylamino propyl) carbodiimide hydrochloride (0.156 g, 0.814 mmol) and DMAP (0.100 g, 0.814 mmol) sequentially at room temperature. The mixture was stirred at room temperature overnight, diluted with DCM and concentrated to provide a residue which was subjected to chromatography to give **42** (0.21 g, 89 %) as a white foam.
¹H NMR (400 MHz, CDCl₃): δ 1.44 (9H, s), 1.65 (1H, m), 1.93 (2H, m), 2.31 (1H, m), 2.76 (1H, d, *J* = 12 Hz), 3.04 (1H, d, *J* = 11.2 Hz), 3.26 (2H, m), 3.38 (1H, m), 3.91 (2H, m), 3.98 (1H, d, *J* = 12 Hz), 4.89 (1H, d, *J* = 11.2 Hz), 5.07 (1H, d, *J =* 11.2 Hz), 5.41 (1H, br s), 7.41 (5H, m), 9.30 (1H, br s).
MS (ES⁺): *m*/*z* [M+H]⁺ calcd for C₂₁H₃₁N₄O₆: 435.22. Found: 435.02.

### Step 2. tert-Butyl {2-[({[(2S,5R)-6-hydroxy-7-oxo-1,6-diazabicyclo[3.2.1]oct-2-yl]carbonyl}amino)oxy]ethyl}carbamate (43)

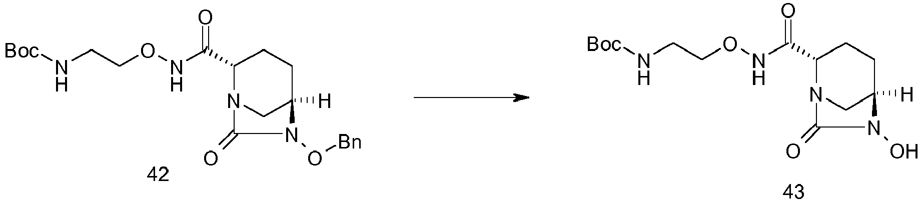

A mixture of *tert-*butyl {2-[({[(2*S*,5*R*)-6-(benzyloxy)-7-oxo-1,6-diazabicyclo[3.2.1]oct-2-yl]carbonyl}amino)oxy]ethyl}carbamate **42** (0.21 g, 0.48 mmol) and Pd/C (0.063 g) in methanol (10 mL) was hydrogenated at 1 atm at room temperature for 3 h. The mixture was filtered through celite pad and concentrated to provide **43** (0.17 g, quant. yield) as a light yellow foam.
¹H NMR (400 MHz, CD₃OD): δ 1.44 (9H, s), 1.75 (1H, m), 1.92 (1H, m), 2.05 (1H, m), 2.23 (1H, m), 3.04 (1H, d, *J* = 12 Hz), 3.12 (2H, m), 3.69 (1H, s), 3.89 (3H, m), 6.74 (1H, br s). 3 protons were not observed in CD₃OD.
MS (ES⁻): *m*/*z* [M-H]⁻ calcd for C₁₄H₂₃N₄O₆: 343.16. Found: 343.00.

### Step 3. tert-Butyl {2-[({[(2S,5R)-7-oxo-6-(sulfooxy)-1,6-diazabicyclo[3.2.1]oct-2-yl]carbonyl}amino)oxy]ethyl}carbamate (44)

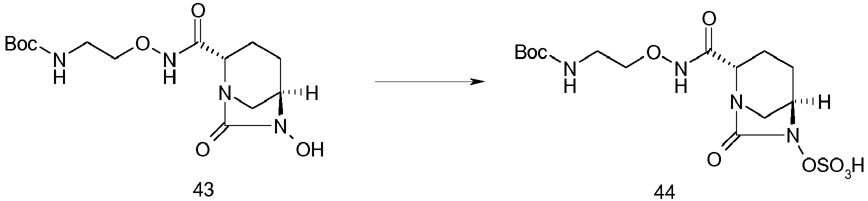

To a mixture of *tert-*butyl {2-[({[(2*S*,5*R*)-6-hydroxy-7-oxo-1,6-diazabicyclo[3.2.1]oct-2-yl]carbonyl}amino)oxy]ethyl}carbamate **43** (0.17 g, 0.49 mmol) in pyridine (7.0 mL) was added sulfur trioxide pyridine complex (0.314 g, 1.98 mmol). The mixture was stirred at room temperature for 23 h and concentrated to provide a residue which was subjected to chromatography to give **44** (0.19 g, 92 %) as a white solid.
¹H NMR (400 MHz, CD₃OD): δ 1.44 (9H, s), 1.80 (1H, m), 1.92 (1H, m), 2.07 (1H, m), 2.20 (1H, m), 3.06 (1H, d, *J* = 12 Hz), 3.28 (2H, m), 3.88 (4H, m), 4.15 (1H, m). 3 protons were not observed in CD₃OD.
MS (ES⁻): *m*/*z* [M-H]⁻ calcd for C₁₄H₂₃N₄O₉S: 423.12. Found: 422.93.

### Step 4. (2S,5R)-N-(2-aminoethoxy)-7-oxo-6-(sulfooxy)-1,6-diazabicyclo [3.2.1]octane-2-carboxamide

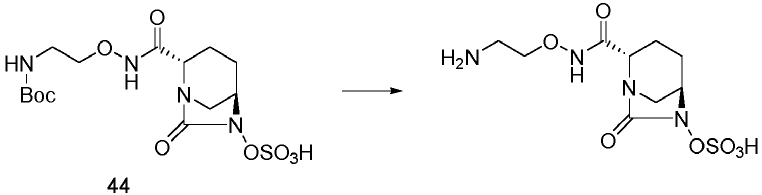

To a mixture of *tert-*butyl {2-[({[(2*S*,5*R*)-7-oxo-6-(sulfooxy)-1,6-diazabicyclo[3.2.1]oct-2-yl]carbonyl}amino)oxy]ethyl}carbamate **44** (0.19 g, 0.45 mmol) in DCM (6.0 mL) was added trifluoroacetic acid (0.30 mL) at 0°C. The mixture was stirred at 0°C for 1 h, concentrated and washed with ether. The white solid was collected by centrifugation. The crude product was purified by preparative HPLC to provide the title compound (44 mg) as a white solid.
¹H NMR (400 MHz, D₂O): δ 1.75 (1H, m), 1.86 (1H, m), 1.95 (1H, m), 2.04 (1H, m), 3.03 (1H, d, *J* = 12 Hz), 3.19 (3H, m), 3.98 (1H, d, *J =* 6.8 Hz), 4.08 (3H, m). 4 protons were not observed in D₂O.
HPLC: 90.18 %.
MS (ES⁻): *m*/*z* [M-H]⁻ calcd for C₉H₁₅N₄O₇S: 323.07. Found: 322.95.

### Example 4

### (2S,5R)-N-(8-Azabicyclo[3.2.1]oct-3-yloxy)-7-oxo-6-(sulfooxy)-1,6-diazabicyclo[3.2.1]octane-2-carboxamide (Compound 27, Table 1)

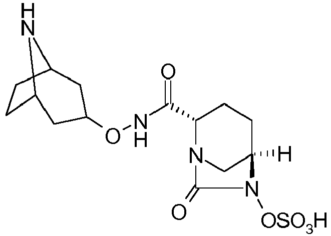

### Step 1. tert-Butyl 3-[({[(2S,5R)-6-(benzyloxy)-7-oxo-1,6-diazabicyclo[3.2.1]oct-2-yl]carbonyl}amino)oxy]-8-azabicyclo[3.2.1]octane-8-carboxylate (46)

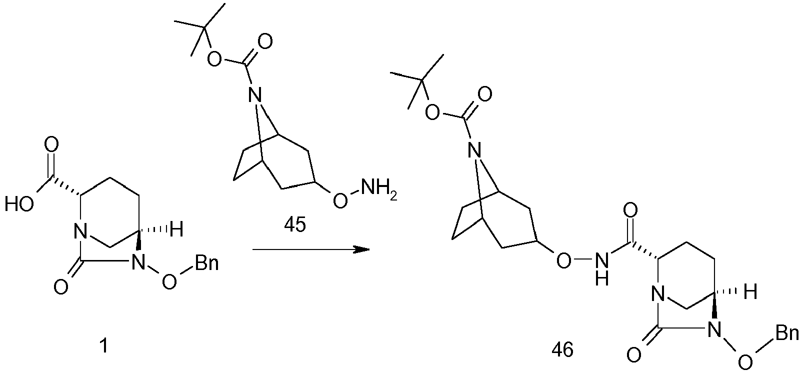

To a solution of (2*S*,5*R*)-6-(benzyloxy)-7-oxo-1,6-diazabicyclo[3.2.1]octane-2-carboxylic acid **1** (0.15 g, 0.54 mmol) in dry DCM (20 mL) were added *tert-*butyl 3-(aminooxy)-8-azabicyclo[3.2.1]octane-8-carboxylate **45** (0.15 g, 0.62 mmol, J. Med. Chem. 2008, 51, 4601-4608), 1-hydroxybenzotriazole (0.11 g, 0.81 mmol) and 1-ethyl-(3-dimethylaminopropyl)carbodiimide hydrochloride (0.16 g, 0.81 mmol) at room temperature. The reaction mixture was stirred at room temperature overnight and concentrated under vacuum. The residue was purified by column chromatography to give *tert-*butyl 3-[({[(2*S*,5*R*)-6-(benzyloxy)-7-oxo-1,6-diazabicyclo[3.2.1]oct-2-yl]carbonyl}amino)oxy]-8-azabicyclo[3.2.1]octane-8-carboxylate **46** (0.26 g, 96 %) as a white solid.
¹H NMR (400 MHz, CDCl₃): δ 1.46 (9H, s), 1.50-1.80 (7H, m), 1.83-2.04 (5H, m), 2.32 (1H, m), 2.72 (1H, d, J = 11.6 Hz), 2.99 (1H, d, J = 11.2 Hz), 3.29 (1H, m), 3.95 (1H, d, J = 7.2 Hz), 4.20-4.38 (2H, m), 4.89 (1H, d, J = 11.2 Hz), 5.05 (1H, d, J = 11.6 Hz), 7.39 (5H, m), 8.90 (1H, br s).

### Step 2. tert-Butyl 3-[({[(2S,5R)-6-hydroxy-7-oxo-1,6-diazabicyclo[3.2.1]oct-2-yl]carbonyl}amino)oxy]-8-azabicyclo[3.2.1]octane-8-carboxylate (47)

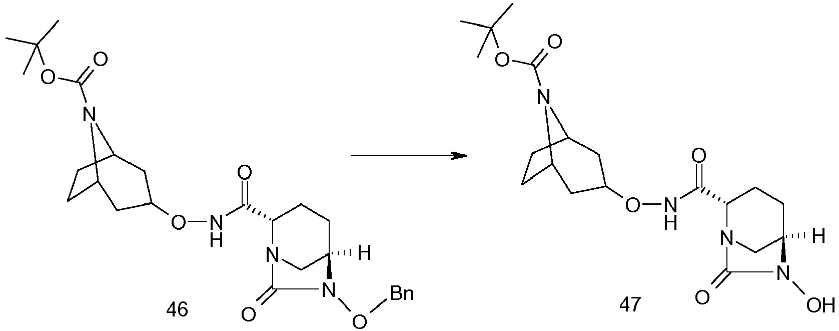

To a solution of *tert-*butyl 3-[({[(2*S*,5*R*)-6-(benzyloxy)-7-oxo-1,6-diazabicyclo[3.2.1]oct-2-yl]carbonyl}amino)oxy]-8-azabicyclo[3.2.1]octane-8-carboxylate **46** (0. 26 g, 0.52 mml) in methanol (20 mL) was added 5% Pd/C (0.3 g). The mixture was hydrogenated under 35 psi hydrogen atmosphere at room temperature for 1 h. The catalyst was filtered out through Celite, and the filtrate was evaporated to give *tert-*butyl 3-[({[(2*S*,5*R*)-6-hydroxy-7-oxo-1,6-diazabicyclo[3.2.1]oct-2-yl]carbonyl}amino)oxy]-8-azabicyclo[3.2.1]octane-8-carboxylate **47** (0.14 g, 66 %) as a white solid.
¹H NMR (400 MHz, CD₃OD): δ 1.48 (9H, s), 1.62-1.76 (4H, m), 1.79-1.85 (1H, m), 1.89-2.00 (3H, m), 2.02-2.11 (3H, m), 2.15-2.20 (1H, m), 3.04-3.17 (2H, m), 3.69 (1H, s), 3.83 (1H, d, J = 7.2 Hz), 4.24 (2H, m), 4.35 (1H, m), 2 protons were not observed in CD₃OD.

### Step 3. tert-Butyl 3-[({[(2S,5R)-7-oxo-6-(sulfooxy)-1,6-diazabicyclo[3.2.1]oct-2-yl]carbonyl}amino)oxy]-8-azabicyclo[3.2.1]octane-8-carboxylate (48)

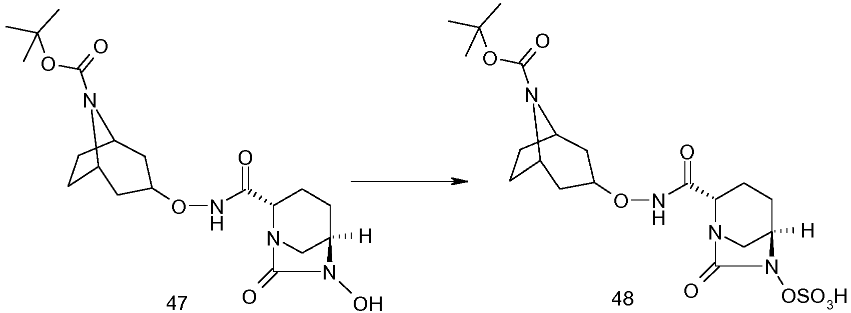

To a solution of *tert-*butyl 3-[({[(2*S*,5*R*)-6-hydroxy-7-oxo-1,6-diazabicyclo[3.2.1]oct-2-yl]carbonyl}amino)oxy]-8-azabicyclo[3.2.1]octane-8-carboxylate **47** (0.14 g, 0.34 mmol) in dry pyridine (6 mL) under nitrogen atmosphere was added sulfur trioxide pyridine complex (0.22 g, 1.36 mmol). The mixture was stirred at room temperature for 20 h, filtered and evaporated. The crude compound was suspended in aqueous acid (a mixture of NaH₂PO₄ and H₃PO₄ to pH 3) and extracted with ethyl acetate (30 mL x 2). The organic extracts were combined, washed with brine, dried over sodium sulfate and evaporated to give *tert-*butyl 3-[({[(2*S*,5*R*)-7-oxo-6-(sulfooxy)-1 ,6-diazabicyclo[3.2.1]oct-2-yl]carbonyl}amino)oxy]-8-azabicyclo[3.2.1]octane-8-carboxylate **48** (0.077 g) which was used in the next step without purification.

### Step 4. (2S,5R)-N-(8-Azabicyclo[3.2.1]oct-3-yloxy)-7-oxo-6-(sulfooxy)-1,6-diazabicyclo[3.2.1]octane-2-carboxamide (Compound 27, Table 1)

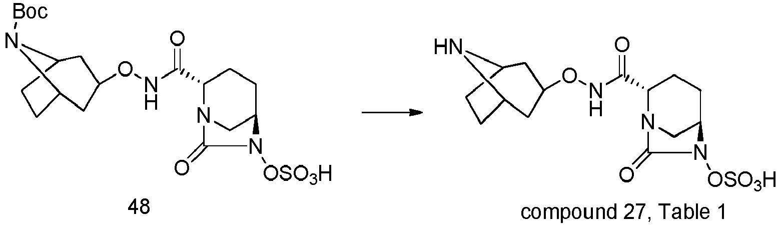

To a solution of *tert-*butyl 3-[({[(2*S*,5*R*)-7-oxo-6-(sulfooxy)-1,6-diazabicyclo[3.2.1]oct-2-yl]carbonyl}amino)oxy]-8-azabicyclo[3.2.1]octane-8-carboxylate **48** (0.077 g, 0.17 mmol) in DCM (7 mL) was added trifluoroacetic acid (0.34 mL, 4.42 mmol) dropwise at 0 °C. The reaction mixture was stirred for 1 h, then evaporated. Ether was added to the residue and the resulting white precipitate was collected by centrifugation. The solid was triturated with acetonitrile (2 x) and the white solid was collected by centrifugation. The white solid was purified by HPLC and freeze-dried to give (2*S*,5*R*)-*N*-(8-azabicyclo[3.2.1]oct-3-yloxy)-7-oxo-6-(sulfooxy)-1,6-diazabicyclo[3.2.1]octane-2-carboxamide **Compound 27 (Table 1)** (0.003 g, 7 %) as a white solid.
¹H NMR (400 MHz, CD₃OD): δ 1.70-2.14 (9H, m), 2.16 - 2.58 (3H, m), 3.07 (1H, d, J = 11.2 Hz), 3.25 (1H, d, J = 11.6 Hz), 3.91 (1H, d, J = 7.2 Hz), 4.00 (2H, m), 4.16 (1H, m), 4.26 (1H, m), 3 protons were not observed in CD₃OD.
HPLC: 81.82 %
MS (ES⁻): m/z [M-H]⁻ = 388.96

### Example 5

### (2S,5R)-N'-acetyl-7-oxo-6-(sulfooxy)-1,6-diazabicyclo[3.2.1]octane-2-carbohydrazide

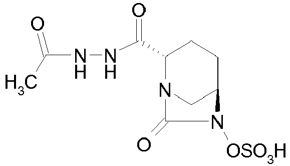

### Step 1. (2S,5R)-N'-Acetyl-6-(benzyloxy)-7-oxo-1,6-diazabicyclo[3.2.1]octane-2-carbohydrazide (174)

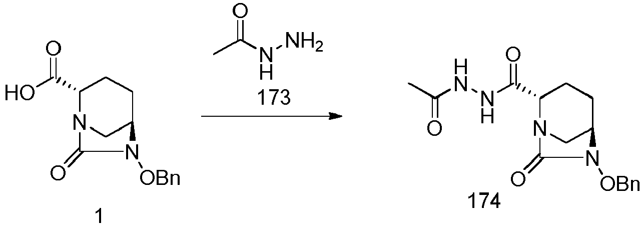

To a mixture of (2*S*,5*R*)-6-(benzyloxy)-7-oxo-1,6-diazabicyclo[3.2.1]octane-2-carboxylic acid **1** (0.200 g, 0.720 mmol, US2005/20572 A1) in DCM (6.0 mL) were added acetohydrazide **173** (0.090 g, 1.085 mmol), 1-hydroxybenzotriazole (0.147 g, 1.085 mmol), 1-ethyl-(3-dimethylamino propyl) carbodiimide hydrochloride (0.208 g, 1.085 mmol) and *N,N-*dimethylaminopyridine sequentially at room temperature. The mixture was stirred at room temperature overnight, diluted with DCM and concentrated to provide a residue, which was subjected to chromatography to give **174** (0.14 g, 60 %) as a white foam.
¹H NMR (400 MHz, CDCl₃): δ 1.60 (1H, m), 1.96 (2H, m), 2.06 (3H, s), 2.34 (1H, m), 3.09 (1H, m), 3.15 (1H, d, *J =* 12 Hz), 3.32 (1H, m), 4.01 (1H, d, *J =* 8.4 Hz), 4.90 (1H, d, *J =* 11.2 Hz), 5.07 (1H, d, *J* = 11.2 Hz), 7.26-7.44 (5H, m), 7.74 (1H, br s), 8.54 (1H, br s).
MS (ES⁺): *m*/*z* [M+H]⁺ calcd for C₁₆H₂₁N₄O₄: 333.16. Found: 333.21.

### Step 2. (2S,5R)-N'-Acetyl-6-hydroxy-7-oxo-1,6-diazabicyclo[3.2.1]octane-2-carbohydrazide (175)

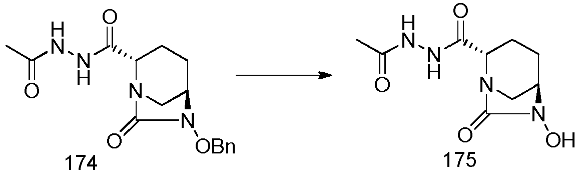

A mixture of (2*S*,5*R*)-*N*'-acetyl-6-(benzyloxy)-7-oxo-1,6-diazabicyclo[3.2.1]octane-2-carbohydrazide **174** (0.14 g, 0.43 mmol) and Pd/C (0.070 g) in methanol (10 mL) was hydrogenated at 1 atm at room temperature for 3 h. The mixture was filtered through Celite pad and concentrated to provide **175** (0.10 g, 95 %) as a white foam.
¹H NMR (400 MHz, CD₃OD): δ 1.71-1.78 (1H, m), 1.88-1.93 (1H, m), 2.04 (3H, s), 2.06-2.09 (1H, m), 2.24-2.29 (1H, m), 3.13 (1H, m), 3.22 (1H, d, *J =* 12 Hz), 3.69 (1H, m), 3.93 (1H, d, *J* = 8.4 Hz). 3 protons were not observed in CD₃OD.
MS (ES⁺): *m*/*z* [M+H]⁺ calcd for C₉H₁₅N₄O₄: 243.11. Found: 243.18.

### Step 3. (2S,5R)-N'-Acety)-7-oxo-6-(sulfooxy)-1,6-diazabicyclo[3.2.1]octane-2-carbohydrazide

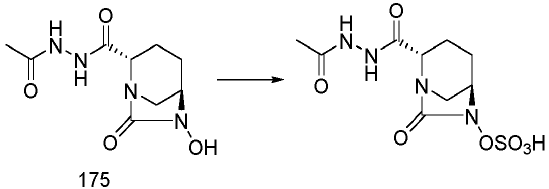

To a mixture of (2S,5*R*)-*N*'-acetyl-6-hydroxy-7-oxo-1,6-diazabicyclo[3.2.1]octane-2-carbohydrazide **175** (0.10 g, 0.41 mmol) in pyridine (5.0 mL) was added sulfur trioxide pyridine complex (0.19 g, 1.24 mmol). The mixture was stirred at room temperature for 23 h and concentrated to provide a residue which was subjected to chromatography to give the title compound (0.040 g, 30 %) as a light yellow solid.
¹H NMR (400 MHz, D₂O): δ 1.64-1.69 (1H, m), 1.76-1.81 (1H, m), 1.88-1.98 (4H, m), 2.03-2.09 (1H, m), 3.03 (1H, d, *J* = 12.0 Hz), 3.20 (1H, m), 4.00 (1H, m), 4.05 (1H, m). 3 protons were not observed in D₂O.
HPLC: 89.2 %
MS (ES⁻): *m*/*z* [M-H]⁻ calcd for C₉H₁₃N₄O₇S: 321.05. Found: 321.05.

The corresponding sodium salt of the title compound was prepared in the following manner:
To a mixture of (2*S*,5*R*)-*N*'-acetyl-6-hydroxy-7-oxo-1,6-diazabicyclo[3.2.1]octane-2-carbohydrazide **175** (0.20 g, 0.78 mmol) in pyridine (10.0 mL) was added sulfur trioxide pyridine complex (0.37 g, 2.35 mmol). The mixture was stirred at room temperature for 24 h and concentrated to provide a residue which was subjected to purification by ion-exchange resin (Dowex50 Na⁺ form, water) and reverse phase column to give sodium salt of the title compound (42 mg) as a white solid.
¹H NMR (400 MHz, D₂O): δ 1.64-1.69 (1H, m), 1.76-1.81 (1H, m), 1.88-1.98 (4H, m), 2.03-2.09 (1H, m), 3.03 (1H, d, *J =* 12.0 Hz), 3.20 (1H, m), 4.00 (1H, m), 4.05 (1H, m). 3 protons were not observed in D₂O.
HPLC: 96.5 %
MS (ES⁻) *m*/*z:* [M-Na]⁻ calcd for C₉H₁₃N₄O₇SNa: 321.05. Found: 321.05.

### Example 6

### (2R,5S)-N'-(methylsulfonyl)-7-oxo-6-(sulfooxy)-1,6-diazabicyclo[3.2.1]octane-2-carbohydrazide (Compound 90, Table 2)

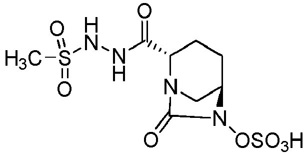

### Step 1. (2R,5R)-6-(benzyloxy)-N'-(methylsulfony)-7-oxo-1,6-diazabicyclo[3.2.1]octane-2-carbohydrazide (188)

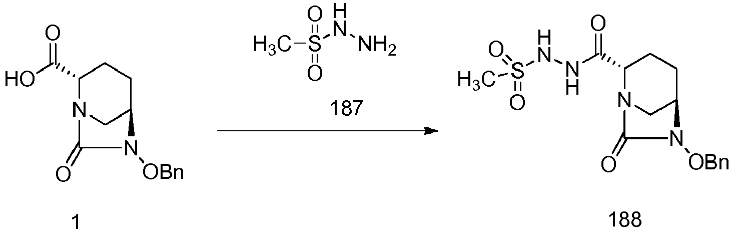

To solution of (2S,5R)-6-(benzyloxy)-7-oxo-1,6-diazabicyclo[3.2.1]octane-2-carboxylic acid 1 (0.67 g, 2.42 mmol) in dry DCM (60 mL) were added methanesulfonohydrazide 187 (0.40 g, 3.63 mmol), 1-hydroxybenzotriazole (0.44 g, 3.63 mmol), 1-ethyl-(3-dimethylaminopropyl)carbodiimide hydrochloride (0.72 g, 3.63 mmol) and 4-(dimethylamino)pyridine (0.44 g, 3.63 mmol) at room temperature. The reaction mixture was stirred at room temperature overnight and concentrated under vacuum. The residue was purified by column chromatography to give (2*R*,5*R*)-6-(benzyloxy)-*N*'-(methylsulfonyl)-7-oxo-1,6-diazabicyclo[3.2.1]octane-2-carbohydrazide **188** (0.37 g, 42%) as a white solid.
¹H NMR (400 MHz, CDCl₃): δ 1.67(1H, m), 2.02 (2H, m), 2.26 (1H, m), 2.79 (1H, d, J = 11.6 Hz), 3.02 (3H, s), 3.09 (1H, d, J = 12.8 Hz), 3.32 (1H, s), 4.12 (1H, d, J = 6.8 Hz ), 4.89 (1H, d, J = 11.2 Hz), 5.03 (1H, d, J = 11.2 Hz), 7.16 (1H, br s), 7.39 (5H, m), 9.08 (1H, br s).

### Step 2. (2R,5R)-6-hydroxy-N'-(methylsulfony)-7-oxo-1,6-diazabicyclo[3.2.1]octane-2-carbohydrazide (189)

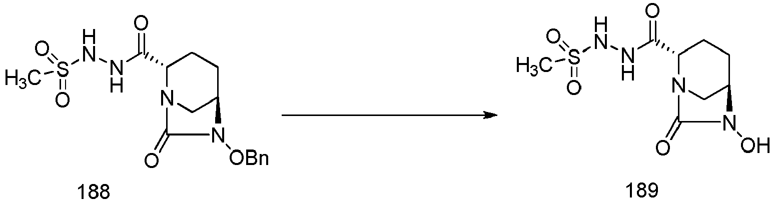

To a solution of (2*R*,5*R*)-6-(benzyloxy)-*N'*-(methylsulfonyl)-7-oxo-1,6-diazabicyclo[3.2.1]octane-2-carbohydrazide **188** (0.37 g, 1.044 mml) in methanol (35 mL) was added 5 % Pd/C (0.40 g). The mixture was hydrogenated under 10 psi hydrogen atmosphere at room temperature for 1 h. The catalyst was filtered through Celite and the filtrate was evaporated to give (2*R*,5*R*)-6-hydroxy-*N*'-(methylsulfonyl)-7-oxo-1,6-diazabicyclo[3.2.1]octane-2-carbohydrazide **189** (0.276 g, 99 %) as a colorless foam.
¹H NMR (400 MHz, CD₃OD): δ 1.78 (1H, m), 1.95 (1H, m), 2.06 (1H, m), 2.24 (1H, m), 3.01 (3H, s), 3.03 (1H, d, J = 12.0 Hz), 3.14 (1H, d, J = 11.6 Hz), 3.69 (1H, s), 3.91 (1H, d, J = 7.6 Hz), 3 protons were not observed in CD₃OD.

### Step 3. (2R,5S)-N'-(methylsulfony)-7-oxo-6-(sulfooxy)-1,6-diazabicyclo[3.2.1]octane-2-carbohydrazide (Compound 90, Table 2)

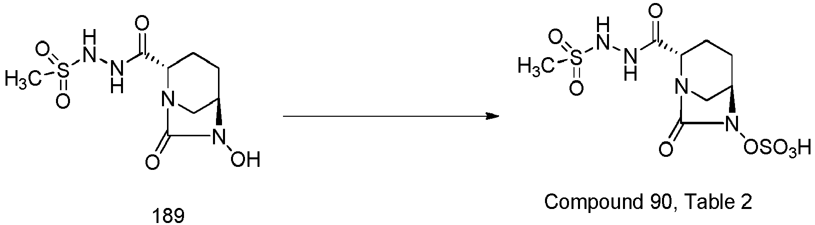

To a solution of (2*R*,5*R*)-6-hydroxy-*N*'-(methylsulfonyl)-7-oxo-1,6-diazabicyclo[3.2.1]octane-2-carbohydrazide **189** (0.276 g, 0.99 mmol) in dry pyridine (18 mL) under nitrogen atmosphere was added sulfur trioxide pyridine complex (0.70 g, 4.37 mmol). The mixture was stirred at room temperature for 20 h, filtered and evaporated. The residue was purified by column chromatography followed by trituration with a mixture of MeOH: DCM: Ether (1:1:1) (4 X) to give (2*R*,5*S*)-*N*'-(methylsulfonyl)-7-oxo-6-(sulfooxy)-1,6-diazabicyclo[3.2.1]octane-2-carbohydrazide **Compound 90 (Table 2)** (0.12 g, 34 %) as a white solid.
¹H NMR (400 MHz, CD₃OD): δ 1.81 (1H, m), 1.96 (1H, m), 2.06 (1H, m), 2.32 (1H, m), 3.05 (1H, d, J = 11.6 Hz), 3.31 (1H, m), 4.01 (1H, d, J = 8.0Hz), 4.16 (1H, s), 3 protons were not observed in CD₃OD.
HPLC: 91.8%
MS (ES⁻) m/z: [M]⁻ = 357

### Example 7

### (2R,5S)-N'-(cyclopropylcarbonyl)-7-oxo-6-(sulfooxy)-1,6-diazabicyclo[3.2.1]octane-2-carbohydrazide

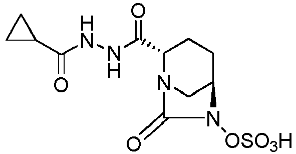

### Step 1. (2R,5S)-6-(benzyloxy)-N'-(cyclopropylcarbonyl)-7-oxo-1,6-diazabicyclo[3.2.1]octane-2-carbohydrazide (197)

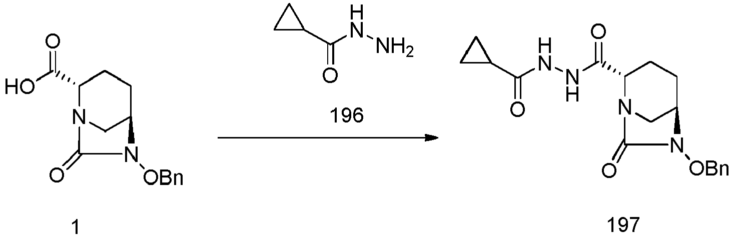

To a solution of (2*S*,5*R*)-6-(benzyloxy)-7-oxo-1,6-diazabicyclo[3.2.1]octane-2-carboxylic acid **1** (0.25 g, 0.90 mmol) in dry DCM (30 mL) were added cyclopropanecarbohydrazide **196** (0.135 g, 1.35 mmol), 1-hydroxybenzotriazole (0.19 g, 1.35 mmol), 1-ethyl-(3-dimethylaminopropyl)carbodiimide hydrochloride (0.26 g, 1.35 mmol) and 4-(dimethylamino)pyridine (0.16 g, 1.35 mmol) at room temperature. The reaction mixture was stirred at room temperature overnight, and concentrated under vacuum. The residue was purified by column chromatography to give (2*R*,5*S*)-6-(benzyloxy)-*N*'-(cyclopropylcarbonyl)-7-oxo-1,6-diazabicyclo[3.2.1]octane-2-carbohydrazide **197** (0.27 g, 84%) as a white solid.
¹H NMR (400 MHz, CDCl₃): δ 0.83 (2H, m),1.04 (2H, m), 1.62 (2H, m), 2.02 (2H, m), 2.34 (1H, m), 3.04 (1H, d, J = 12.0 Hz), 3.15 (1H, d, J = 12.0 Hz), 3.29 (1H, s), 4.00 (1H, d, J = 7.6 Hz), 4.89 (1H, d, J = 11.2 Hz), 5.03 (1H, d, J = 11.2 Hz), 7.38 (5H, m), 8.29 (1H, br s), 8.57 (1H, br s).

### Step 2. (2R,5S)-N'-(cyclopropylcarbonyl)-6-hydroxy-7-oxo-1,6-diazabicyclo[3.2.1]octane-2-carbohydrazide (198)

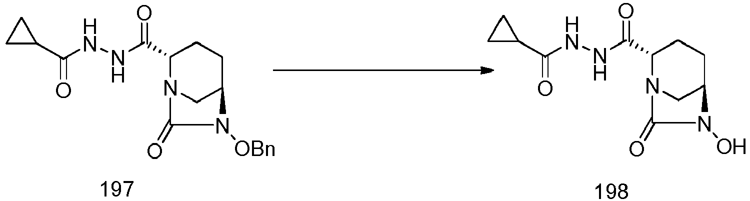

To a solution of (2*R*,5*S*)-6-(benzyloxy)-*N*-(cyclopropylcarbonyl)-7-oxo-1,6-diazabicyclo[3.2.1]octane-2-carbohydrazide **197** (0. 27 g, 0.75 mml) in methanol (20 mL) was added 5% Pd/C (0.30 g). The mixture was hydrogenated under 10 psi hydrogen atmosphere at room temperature for 1 h. The catalyst was filtered out through Celite, and the filtrate was evaporated to give (2*R*,5*S*)-*N'-*(cyclopropylcarbonyl)-6-hydroxy-7-oxo-1,6-diazabicyclo[3.2.1]octane-2-carbohydrazide **198** (0.20 g, 98%) as a colorless foam.
¹H NMR (400 MHz, CD₃OD): δ 0.86 (2H, m), 0.91 (2H, m), 1.65 (1H,m), 1.76 (1H, m), 1.94 (1H, m), 2.04 (1H, m), 2.26 (1H, m), 3.13 (1H, d, J = 13.2 Hz), 3.23 (1H, d, J = 12.0 Hz), 3.70 (1H, s), 3.94 (1H, d, J = 7.2 Hz), 3 protons were not observed in CD₃OD.

### Step 3. (2R,5S)-N-(cyclopropylcarbonyl)-7-oxo-6-(sulfooxy)-1,6-diazabicyclo[3.2.1 ]octane-2-carbohydrazide

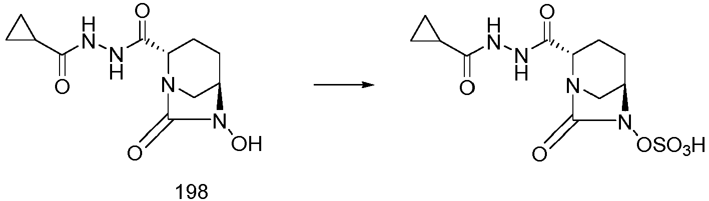

To a solution of (2*R*,5*S*)-*N'-*(cyclopropylcarbonyl)-6-hydroxy-7-oxo-1,6-diazabicyclo[3.2.1]octane-2-carbohydrazide **198** (0.20 g, 0.75 mmol) in dry pyridine (7 mL) under nitrogen atmosphere was added sulfur trioxide pyridine complex (0.70 g, 4.40 mmol). The mixture was stirred at room temperature for 20 h, filtered and evaporated. The residue was purified first by column chromatography followed by HPLC on a prep-X Bridge-30x100 mm column and freeze-dried to give (2*R*,5*S*)-*N'*-(cyclopropylcarbonyl)-7-oxo-6-(sulfooxy)-1,6-diazabicyclo[3.2.1]octane-2-carbohydrazide (0.035 g) as a grey solid.
¹H NMR (400 MHz, CD₃OD): δ 0.83 (2H, m), 0.90 (2H, m), 1.66 (1H, m), 1.78 (1H, m), 1.94 (1H, m), 2.06 (1H, m), 2.28 (1H, m), 3.30 (2H, m), 4.01 (1H, d, J = 7.6 Hz), 4.14 (1H, s), 3 protons were not observed in CD₃OD.
HPLC: 98.4%
MS (ES⁻) m/z: [M]⁻= 347

### Example 8

### (2S,5R)-7-oxo-N'-propanoyl-6-(sulfooxy)-1,6-diazabicyclo[3.2.1]octane-2-carbohydrazide

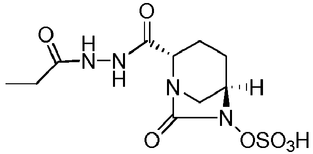

### Step 1. (2S,5R)-6-(benzy)oxy)-7-oxo-N'-propanoyl-1,6-diazabicyclo[3.2.1]octane-2-carbohydrazide (203)

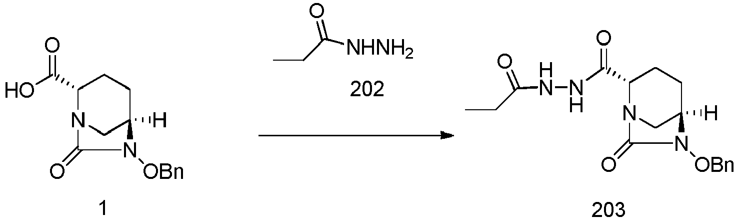

To a mixture of (2*S*,5*R*)-6-(benzyloxy)-7-oxo-1,6-diazabicyclo[3.2.1]octane-2-carboxylic acid **1** (0.250 g, 0.905 mmol) in DCM (15.0 mL) were added propanehydrazide **202** (0.120 g, 1.358 mmol), 1-hydroxybenzotriazole (0.186 g, 1.358 mmol) and 1-ethyl-(3-dimethylamino propyl) carbodiimide hydrochloride (0.260 g, 1.358 mmol) sequentially at room temperature. The mixture was stirred at room temperature overnight, diluted with DCM and concentrated to provide a residue which was subjected to chromatography to give **203** (0.31 g, 99 %) as a white foam.
¹H NMR (400 MHz, CDCl₃): δ 1.20 (3H, t, *J =* 7.4 Hz), 1.62 (1H, m), 1.98 (2H, m), 2.12 (1H, m), 2.38 (2H, m), 3.10 (1H, d, *J =* 12.1 Hz), 3.19 (1H, d, *J =* 12.1 Hz), 3.30 (1H, s), 3.90 (2H, br s), 4.02 (1H, d, *J* = 7.4 Hz), 4.90 (1H, d, *J* = 11.3 Hz), 5.05 (1H, d, *J* = 11.3 Hz), 7.42 (5H, m).
MS (ES⁺) *m*/*z:* [M+H]⁺ calcd for C₁₁H₂₃N₄O₄: 347.2. Found: 347.2.

### Step 2. (2S,5R)-6-hydroxy-7-oxo-N'-propanoyl-1,6-diazabicyclo[3.2.1]octane-2-carbohydrazide (204)

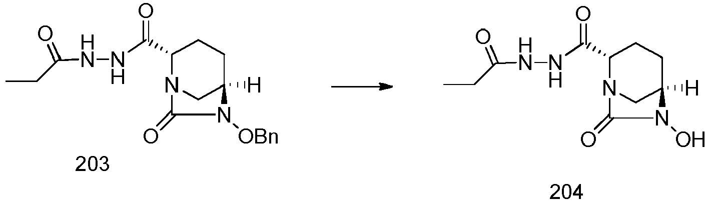

A mixture of (2*S*,5*R*)-6-(benzyloxy)-7-oxo-*N*'-propanoyl-1,6-diazabicyclo[3.2.1]octane-2-carbohydrazide **203** (0.31 g, 0.89 mmol) and Pd/C (0.12 g) in methanol (20 mL) was hydrogenated at 1 atm at room temperature for 16 h. The mixture was filtered through Celite and concentrated to provide **204** (0.24 g, quant. yield) as a white foam.
¹H NMR (400 MHz, CD₃OD): δ 1.18 (3H, t, *J =* 7.4 Hz), 1.75 (1H, m), 1.85 (1H, m), 1.96 (1H, m), 2.08 (1H, m), 2.30 (2H, m), 3.18 (1H, m), 3.30 (1H, m), 3.70 (1H, br s), 3.95 (1H, d, *J =* 7.4 Hz). 3 protons were not observed in CD₃OD.
MS (ES⁻) m/z: [M-H]⁻ calcd for C₁₀H₁₄N₄O₄: 255.1. Found: 255.0.

### Step 3. (2S,5R)-7-oxo-N'-propanoyl-6-(sulfooxy)-1,6-diazabicyclo[3.2.1]octane-2-carbohydrazide (Compound 17, Table 2)

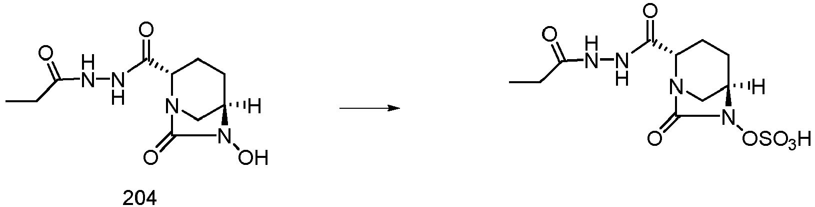

To a mixture of (2*S*,5*R*)-6-hydroxy-7-oxo-*N*'-propanoyl-1,6-diazabicyclo[3.2.1]octane-2-carbohydrazide **204** (0.24 g, 0.93 mmol) in pyridine (5.0 mL) was added sulfur trioxide pyridine complex (0.44 g, 2.81 mmol). The mixture was stirred at room temperature for 23 h and concentrated to provide a residue which was subjected to chromatography and HPLC purification to give the title compound (0.053 g, 17 %) as a white solid.
¹H NMR (400 MHz, D₂O): δ 0.98 (3H, t, *J* = 7.8 Hz), 1.65 (1H, m), 1.80 (1H, m), 1.95 (1H, m), 2.05 (1H, m), 2.18 (2H, q, *J* = 7.8 Hz), 3.05 (1H, d, J = 12.1 Hz), 3.18 (1H, d, *J* = 12.9 Hz), 4.00 (1H, d, *J* = 7.8 Hz), 4.05 (1H, m). 3 protons were not observed in D₂O.
HPLC: 97.12 %
MS (ES⁻) *m*/*z:* [M-H]⁻ calcd for C₁₀H₁₅N₄O₇S: 335.1. Found: 335.0.

### Example 9

### (2R,5S)-N'-(azetidin-3-ylcarbonyl)-7-oxo-6-(sulfooxy)-1,6-diazabicyclo[3.2.1]octane-2-carbohydrazide

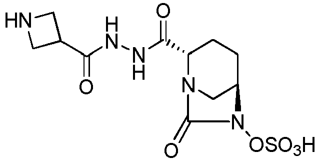

### Step 1. tert-butyl 3-[(2-{[(2R,5S)-6-(benzyloxy)-7-oxo-1,6-diazabicyclo[3.2.1]oct-2-yl]carbonyl}hydrazinyl)carbonyl]azetidine-1-carboxylate (260)

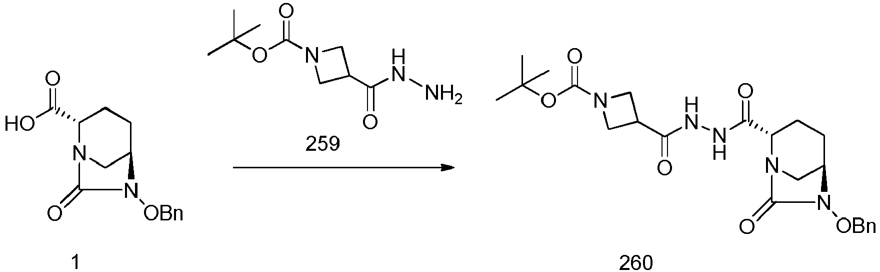

To a solution of (2*S*,5*R*)-6-(benzyloxy)-7-oxo-1,6-diazabicyclo[3.2.1]octane-2-carboxylic acid **1** (0.25 g, 0.90 mmol) in dry DCM (30 mL) were added *tert-butyl* 3-(hydrazinylcarbonyl)azetidine-1-carboxylate **259** (0.29 g, 1.35 mmol), 1-hydroxybenzotriazole (0.19 g, 1.35 mmol), 1-ethyl-(3-dimethylaminopropyl)carbodiimide hydrochloride (0.26 g, 1.35 mmol) and 4-(dimethylamino)pyridine (0.16 g, 1.35 mmol) at room temperature. The reaction mixture was stirred at room temperature overnight and concentrated under vacuum. The residue was purified by column chromatography to give *tert-*butyl 3-[(2-{[(2*R*,5*S*)-6-(benzyloxy)-7-oxo-1,6-diazabicyclo[3.2.1]oct-2-yl]carbonyl}hydrazinyl)carbonyl]azetidine-1-carboxylate **260** (0.35 g, 81%) as a white solid.
¹H NMR (400 MHz, CDCl₃): δ 1.38 (9H, s), 1.63 (1H, m), 1.99 (2H, m), 2.31 (1H, m), 3.15 (2H, m), 3.28 (2H, s), 4.00 (1H, d, J= 7.6 Hz), 4.10 (4H, m), 4.90 (1H, d, J = 11.2 Hz), 5.04 (1H, d, J = 11.6 Hz), 7.39 (5H, m), 7.86 (1H, br s), 8.54 (1H, br s).

### Step 2. tert-butyl 3-[(2-{[(2R,5S)-6-hydroxy-7-oxo-1,6-diazabicyclo[3.2.1]oct-2-yl]carbonyl}hydrazinyl)carbonyl]azetidine-1-carboxylate (261)

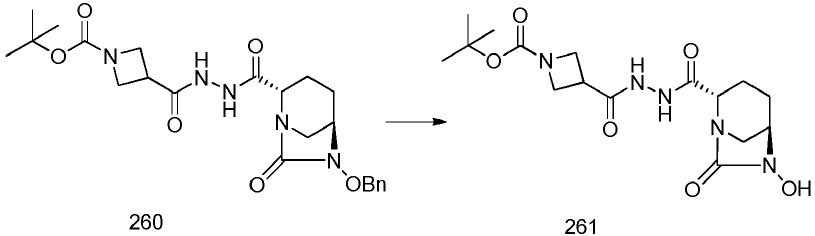

To a solution of *tert-*butyl 3-[(2-{[(2*R*,5*S*)-6-(benzyloxy)-7-oxo-1,6-diazabicyclo[3.2.1]oct-2-yl]carbonyl}hydrazinyl)carbonyl]azetidine-1-carboxylate **260** (0. 35 g, 0.74 mml) in methanol (25 mL) was added 5% Pd/C (0.40 g). The mixture was hydrogenated under 10 psi hydrogen atmosphere at room temperature for 1 h. The catalyst was filtered out through Celite, and the filtrate was evaporated to give *tert-*butyl 3-[(2-{[(2*R*,5*S*)-6-hydroxy-7-oxo-1,6-diazabicyclo[3.2.1]oct-2-yl]carbonyl}hydrazinyl)carbonyl]azetidine-1-carboxylate **261** (0.25 g, 88%) as a white foam.
¹H NMR (400 MHz, CD₃OD): δ 1.44 (9H, s), 1.76 (1H, m), 1.91 (1H, m), 2.05 (1H, m), 2.26 (1H, m), 3.15 (1H, m), 3.25 (1H, m), 3.40 (1H, m), 3.71 (1H, s), 3.95 (1H, d, J = 7.2 Hz), 4.08 (4H, m), 3 protons were not observed in CD₃OD.

### Step 3. tert-butyl 3-[(2-{[(2R,5S)-7-oxo-6-(sulfooxy)-1,6-diazabicyclo[3.2.1]oct-2-yl]carbonyl}hydrazinyl)carbonyl]azetidine-1-carboxylate (262)

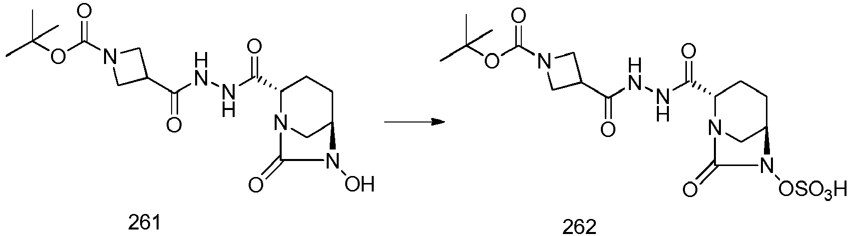

To a solution of *tert*-butyl 3-[(2-{[(2*R*,5*S*)-6-hydroxy-7-oxo-1,6-diazabicyclo[3.2.1]oct-2-yl]carbonyl}hydrazinyl)carbonyl]azetidine-1-carboxylate **261** (0.25 g, 0.65 mmol) in dry pyridine (10 mL) under nitrogen atmosphere was added sulfur trioxide pyridine complex (0.60 g, 3.80 mmol). The mixture was stirred at room temperature for 20 h, filtered and evaporated. The residue was purified first by column chromatography to give *tert*-butyl 3-[(2-{[(2*R*,5*S*)-7-oxo-6-(sulfooxy)-1 ,6-diazabicyclo[3.2.1]oct-2-yl]carbonyl}hydrazinyl)carbonyl]azetidine-1-carboxylate **262** (0.18 g, 60%) as a white solid.
¹H NMR (400 MHz, CD₃OD): δ 1.30 (9H, s), 1.81 (1H, m), 1.94 (1H, m), 2.05 (1H, m), 2.26 (1H, m), 3.30 (2H, m), 3.41 (1H, m), 4.04 (5H, m), 4.16 (1H, s), 3 protons were not observed in CD₃OD.

### Step 4. (2R,5S)-N'-(azetidin-3-ylcarbonyl)-7-oxo-6-(sulfooxy)-1,6-diazabicyclo[3.2.1]octane-2-carbohydrazide

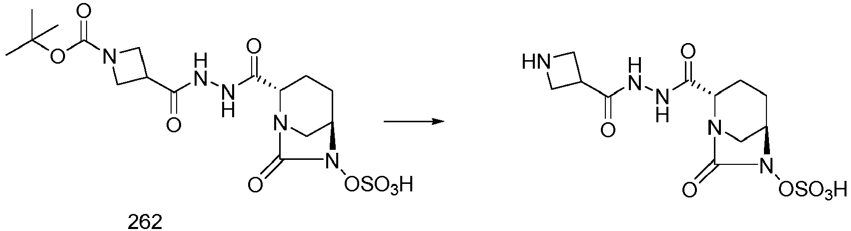

To a solution *tert*-butyl 3-[(2-{[(2*R*,5*S*)-7-oxo-6-(sulfooxy)-1,6-diazabicyclo[3.2.1]oct-2-yl]carbonyl}hydrazinyl)carbonyl]azetidine-1-carboxylate **262** (0.18 g, 0.39 mmol) in DCM (18 mL) was added trifluoroacetic acid (1.45 mL, 18.79 mmol) dropwise at 0 °C. The reaction mixture was stirred for 1 h then evaporated. Ether was added to the residue and the resulting white precipitate was collected by centrifugation. The solid was triturated with a mixture of MeOH:ether (1:5, 6X) and the white solid was collected by centrifugation to give (2*R*,5*S*)-*N'-*(azetidin-3-ylcarbonyl)-7-oxo-6-(sulfooxy)-1,6-diazabicyclo[3.2.1]octane-2-carbohydrazide (0.04 g, 28%) as a white solid.
¹H NMR (400 MHz, D₂O): δ 1.66 (1H, m), 1.77 (1H, m), 1.90 (1H, m), 2.04 (1H, m), 3.03 (1H, d, J= 12.4 Hz), 3.17 (1H, m), 3.65 (1H, m), 4.02 (2H, m), 4.12 (4H, m), 4 protons were not observed in D₂O.
HPLC 92.40%
MS (ES⁻) m/z: [M]⁻= 362

### Antibacterial activity and synergistic activity:

Compounds herein disclosed alone, ceftazidime alone, meropenem alone, aztreonam alone and as a combination with these antibiotics were tested for minimum inhibitory concentration (MIC, µg/mL) against bacteria listed in Tables 3 - 9. In the Tables 10 - 12, compounds herein disclosed were tested in combination with various antibiotics against metallo β-lactamase producing bacteria.

**Table 3: Synergy of the inhibitor Ex. 1 (4 µg/mL) in combination with antibiotics**

| | | | | | | | | |
|---|---|---|---|---|---|---|---|---|
| 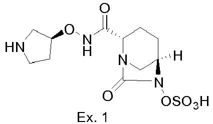 | | | | | | | | |

| **Organism** | **Enzyme** | **Ex. 1 Alone** | **Meropenem** | **Meropenem + Ex. 1 (4 µg/mL)** | **Ceftazidime** | **Ceftazidime + Ex.1 (4 µg/mL)** | **Aztreonam** | **Aztreonam + Ex. 1 (4 mg/mL)** |
|---|---|---|---|---|---|---|---|---|
| E. coli ATCC 25922 | Wt | 8 | 0.06 | ≤.031 | 0.25 | ≤0.25 | 0.25 | ≤.062 |
| A. baumanii JMI 2659 | Ges-14 (Cpase) | >16 | 64 | 2 | 512 | >16 | 512 | >4 |
| E.coli JMI 4103 | KPC-2, Tem-1, CMY-Type | 16 | 64 | ≤.031 | >512 | 8 | >512 | >4 |
| E. coli JMI 4080 | Tem-10 | 8 | ≤1 | ≤.031 | 64 | 1 | 64 | 1 |
| E. coli JMI 2692 | NDM-1, TEM-1, CTX-M-15 | 4 | 256 | ≤.031 | >512 | ≤0.25 | >512 | 0.25 |
| E. coli JMI 2671 | VIM-19 (Cpase) | 4 | 64 | 2 | 64 | 2 | 64 | ≤.062 |
| E. coli JMI 2665 | CMY-2 (Plasmid Cpase) | 4 | ≤1 | ≤.031 | 8 | ≤0.25 | 8 | ≤.062 |
| K.pneumo JMI 4109 | SHV-1, SHV-12 | 4 | ≤1 | ≤.031 | 8 | ≤0.25 | 8 | ≤.062 |
| K. pneumo JMI 2673 | CTX-M14 (ESBL) | >16 | ≤1 | ≤.031 | 32 | ≤0.25 | 32 | ≤.062 |
| K. pneumo JMI 2674 | CTX-M15 (ESBL) | >16 | ≤1 | 0.062 | 512 | ≤0.25 | 512 | ≤.062 |
| K. pneumo JMI 4088 | KPC-3 (Cpase) | 4 | 512 | 0.062 | >512 | ≤0.25 | >512 | ≤.062 |
| K. pneumo JMI 4106 | KPC-3, TEM-1, SHV-12, SHV-141 | 16 | 128 | ≤.031 | >512 | ≤0.25 | >512 | 0.25 |
| K. pneumo JMI 2693 | NDM-1 (Cpase) | >16 | 8 | 0.062 | >512 | ≤0.25 | >512 | 0.125 |
| K. pneumo JMI 2697 | IMP-4 (Cpase) | 16 | 64 | >2 | >512 | 16 | >512 | 0.25 |
| K. pneumo JMI 2681 | Oxa-48 (Cpase) | >16 | 64 | >2 | 512 | 4 | 512 | 4 |
| K. pneumo JMI 2699 | VIM-1, CTX-M3 | 8 | 32 | 2 | >512 | 16 | >512 | 0.5 |
| P. aerug JMI 2686 | KPC-2 (Cpase) | >16 | 512 | >2 | 512 | 16 | 512 | >4 |
| P. aerug JMI 149 | Bla+++D+ | >16 | 4 | 0.5 | 256 | 4 | 256 | >4 |
| E. cloacae JMI 36 | P99 | >16 | ≤1 | 0.062 | 128 | 1 | 128 | 0.25 |
| E. coli JMI 10767 | Wt | 8 | ≤1 | ≤.031 | ≤1 | ≤0.25 | ≤1 | ≤.062 |
| E. coli JMI 10768 | CTX-M15 (Weak ESBL) | 8 | ≤1 | ≤.031 | 8 | ≤0.25 | 8 | ≤.062 |
| E. coli JMI 10770 | CTX-M15 (Hyper ESBL) | 8 | ≤1 | ≤.031 | 64 | ≤0.25 | 64 | ≤.062 |
| E. coli JMI 11103 | CTX-M15 (Intermediate ESBL) | 8 | ≤1 | ≤.031 | 32 | ≤0.25 | 32 | ≤.062 |

**Table 4: Synergy of the inhibitor Ex. 2 (4 µg/mL) in combination with antibiotics**

| | | | | | | | | |
|---|---|---|---|---|---|---|---|---|
| 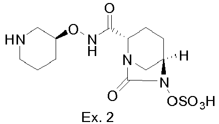 | | | | | | | | |

| **Organism** | **Enzyme** | **Ex. 2 Alone** | **Meropenem** | **Meropenem + Ex. 2 (4 µg/mL)** | **Ceftazidime** | **Ceftazidime + Ex. 2 (4 µg/mL)** | **Aztreonam** | **Aztreonam + Ex. 2 (4 mg/mL)** |
|---|---|---|---|---|---|---|---|---|
| E. coli ATCC 25922 | Wt | 1 | 0.06 | ≤.031 | 0.25 | ≤0.25 | 0.25 | ≤.062 |
| A. baumanii JMI 2659 | Ges-14 (Cpase) | >16 | 64 | >2 | 512 | >16 | 512 | >4 |
| E.coli JMI 4103 | KPC-2, Tem-1, CMY-Type | 8 | 64 | ≤.031 | >512 | 8 | >512 | >4 |
| E. coli JMI 4080 | Tem-10 | 4 | ≤1 | ≤.031 | 64 | ≤0.25 | 64 | ≤.062 |
| E. coli JMI 2692 | NDM-1, TEM-1, CTX-M-15 | ≤0.5 | 256 | ≤.031 | >512 | ≤0.25 | >512 | ≤.062 |
| E. coli JMI 2671 | VIM-19 (Cpase) | >16 | 64 | 0.125 | 64 | 0.5 | 64 | ≤.062 |
| E. coli JMI 2665 | CMY-2 (Plasmid Cpase) | ≤0.5 | ≤1 | ≤.031 | 8 | ≤0.25 | 8 | ≤.062 |
| K.pneumo JMI 4109 | SHV-1, SHV-12 | 16 | ≤1 | ≤.031 | 8 | ≤0.25 | 8 | ≤.062 |
| K. pneumo JMI 2673 | CTX-M14 (ESBL) | >16 | ≤1 | ≤.031 | 32 | ≤0.25 | 32 | ≤.062 |
| K. pneumo JMI 2674 | CTX-M15 (ESBL) | >16 | ≤1 | 0.125 | 512 | ≤0.25 | 512 | 0.125 |
| K. pneumo JMI 4088 | KPC-3 (Cpase) | 1 | 512 | 0.062 | >512 | ≤0.25 | >512 | 0.5 |
| K. pneumo JMI 4106 | KPC-3, TEM-1, SHV-12, SHV-141 | >16 | 128 | ≤.031 | >512 | ≤0.25 | >512 | ≤.062 |
| K. pneumo JMI 2693 | NDM-1 (Cpase) | 4 | 8 | ≤.031 | >512 | ≤0.25 | >512 | ≤.062 |
| K. pneumo JMI 2697 | IMP-4 (Cpase) | 4 | 64 | ≤.031 | >512 | 0.5 | >512 | ≤.062 |
| K. pneumo JMI 2681 | Oxa-48 (Cpase) | >16 | 64 | >2 | 512 | 1 | 512 | **2** |
| K. pneumo JMI 2699 | VIM-1, CTX-M3 | 4 | 32 | 0.062 | >512 | ≤0.25 | >512 | ≤.062 |
| P. aerug JMI 2686 | KPC-2 (Cpase) | >16 | 512 | >2 | 512 | 16 | 512 | >4 |
| P. aerug JMI 149 | Bla+++D+ | >16 | 4 | 0.5 | 256 | 4 | 256 | >4 |
| E. cloacae JMI 36 | P99 | >16 | ≤1 | 0.125 | 128 | ≤0.25 | 128 | 0.125 |
| E. coli JMI 10767 | Wt | 4 | ≤1 | ≤.031 | ≤1 | ≤0.25 | ≤1 | ≤.062 |
| E. coli JMI 10768 | CTX-M15 (Weak ESBL) | 4 | ≤1 | ≤.031 | 8 | ≤0.25 | 8 | ≤.062 |
| E. coli JMI 10770 | CTX-M15 (Hyper ESBL) | 4 | ≤1 | ≤.031 | 64 | ≤0.25 | 64 | ≤.062 |
| E. coli JMI 11103 | CTX-M15 (Intermediate ESBL) | 4 | ≤1 | ≤.031 | 32 | ≤0.25 | 32 | ≤.062 |

**Table 5: Synergy of the inhibitor Ex. 3 (4 µg/mL) in combination with antibiotics**

| | | | | | | | | |
|---|---|---|---|---|---|---|---|---|
| 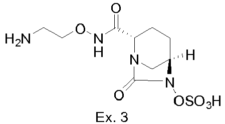 | | | | | | | | |

| **Organism** | **Enzyme** | **Ex. 3 Alone** | **Meropenem** | **Meropenem + Ex. 3 (4 ng/mL)** | **Ceftazidime** | **Ceftazidime + Ex. 3 (4 µg/mL)** | **Aztreonam** | **Aztreonam + Ex. 3 (4 mg/mL)** |
|---|---|---|---|---|---|---|---|---|
| E. coli ATCC 25922 | Wt | 2 | 0.06 | ≤0.0312 | 0.25 | ≤0.25 | 0.25 | ≤0.062 |
| A. baumanii JMI 2659 | Ges-14 (Cpase) | >16 | 64 | 2 | 512 | >16 | 512 | >4 |
| E.coli JMI 4103 | KPC-2, Tem-1, CMY-Type | 4 | 64 | ≤0.0312 | >512 | ≤0.25 | >512 | ≤0.062 |
| E. coli JMI 4080 | Tem-10 | 2 | ≤1 | ≤0.0312 | 64 | ≤0.25 | 64 | ≤0.062 |
| E. coli JMI 2692 | NDM-1, TEM-1, CTX-M-15 | 2 | 256 | ≤0.0312 | >512 | ≤0.25 | >512 | ≤0.062 |
| E. coli JMI 2671 | VIM-19 (Cpase) | 2 | 64 | ≤0.0312 | 64 | ≤0.25 | 64 | ≤0.062 |
| E. coli JMI 2665 | CMY-2 (Plasmid Cpase) | 2 | ≤1 | ≤0.0312 | 8 | ≤0.25 | 8 | ≤0.062 |
| K.pneumo JMI 4109 | SHV-1, SHV-12 | 2 | ≤1 | ≤0.0312 | 8 | ≤0.25 | 8 | ≤0.062 |
| K. pneumo JMI 2673 | CTX-M14 (ESBL) | >16 | ≤1 | ≤0.0312 | 32 | ≤0.25 | 32 | 0.125 |
| K. pneumo JMI 2674 | CTX-M15 (ESBL) | >16 | ≤1 | 0.125 | 512 | ≤0.25 | 512 | 0.5 |
| K. pneumo JMI 4088 | KPC-3 (Cpase) | 2 | 512 | 0.25 | >512 | ≤0.25 | >512 | 1 |
| K. pneumo JMI 4106 | KPC-3, TEM-1, SHV-12, SHV-141 | 2 | 128 | ≤0.0312 | >512 | ≤0.25 | >512 | ≤0.062 |
| K. pneumo JMI 2693 | NDM-1 (Cpase) | 8 | 8 | ≤0.0312 | >512 | ≤0.25 | >512 | 0.25 |
| K. pneumo JMI 2697 | IMP-4 (Cpase) | 8 | 64 | 0.25 | >512 | 0.5 | >512 | 0.25 |
| K. pneumo JMI 2681 | Oxa-48 (Cpase) | >16 | 64 | 0.5 | 512 | ≤0.25 | 512 | 1 |
| K. pneumo JMI 2699 | VIM-1, CTX-M3 | 4 | 32 | ≤0.0312 | >512 | ≤0.25 | >512 | ≤0.062 |
| P. aerug JMI 2686 | KPC-2 (Cpase) | >16 | 512 | >2 | 512 | 16 | 512 | >4 |
| P. aerug JMI 149 | Bla+++D+ | >16 | 4 | 0.25 | 256 | 4 | 256 | >4 |
| E. cloacae JMI 36 | P99 | >16 | ≤1 | 0.062 | 128 | 0.5 | 128 | >4 |
| E. coli JMI 10767 | Wt | 2 | ≤1 | ≤0.0312 | ≤1 | ≤0.25 | ≤1 | ≤0.062 |
| E. coli JMI 10768 | CTX-M15 (Weak ESBL) | 4 | ≤1 | 0.062 | 8 | ≤0.25 | 8 | ≤0.062 |
| E. coli JMI 10770 | CTX-M15 (Hyper ESBL) | >16 | ≤1 | 0.062 | 64 | 2 | 64 | 8 |
| E. coli JMI 11103 | CTX-M15 (Intermediate ESBL) | 4 | ≤1 | 0.062 | 32 | 0.5 | 32 | 0.5 |

**Table 6: Synergy of the inhibitor Ex. 5 (4 µg/mL) in combination with antibiotics**

| | | | | | | | | |
|---|---|---|---|---|---|---|---|---|
| 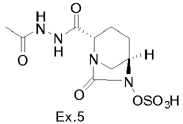 | | | | | | | | |

| **Organism** | **Enzyme** | **Ex. 5 Alone** | **Meropenem** | **Meropenem + Ex. 5 (4 µg/mL)** | **Ceftazidime** | **Ceftazidime + Ex. 5 (4 µg/mL)** | **Aztreonam** | **Aztreonam + Ex. 5 (4 µg/mL)** |
|---|---|---|---|---|---|---|---|---|
| E. coli ATCC 25922 | Wt | **2** | 0.06 | ≤0.031 | 0.25 | ≤0.25 | 0.125 | ≤0.062 |
| A. baumanii JMI 2659 | Ges-14 (Cpase) | >16 | 64 | >2 | 512 | >16 | 256 | >4 |
| E.coli JMI 4103 | KPC-2, Tem-1, CMY-Type | 8 | 64 | ≤0.031 | >512 | ≤0.25 | 256 | ≤0.062 |
| E. coli JMI 4080 | Tem-10 | 2 | ≤1 | ≤0.031 | 64 | ≤0.25 | 125 | ≤0.062 |
| E. coli JMI 2692 | NDM-1, TEM-1, CTX-M-15 | 2 | 256 | ≤0.031 | >512 | ≤0.25 | 256 | ≤0.062 |
| E. coli JMI 2671 | VIM-19 (Cpase) | 16 | 64 | 0.25 | 64 | 2 | 16 | ≤0.062 |
| E. coli JMI 2665 | CMY-2 (Plasmid Cpase) | 2 | ≤1 | ≤0.031 | 8 | ≤0.25 | 64 | ≤0.062 |
| K.pneumo JMI 4109 | SHV-1, SHV-12 | >16 | ≤1 | ≤0.031 | 8 | ≤0.25 | ≤1 | ≤0.062 |
| K. pneumo JMI 2673 | CTX-M14 (ESBL) | >16 | ≤1 | ≤0.031 | 32 | ≤0.25 | 8 | ≤0.062 |
| K. pneumo JMI 2674 | CTX-M15 (ESBL) | >16 | ≤1 | 0.062 | 512 | ≤0.25 | 32 | 0.125 |
| K. pneumo JMI 4088 | KPC-3 (Cpase) | >16 | 512 | 0.125 | >512 | ≤0.25 | 512 | 0.25 |
| K. pneumo JMI 4106 | KPC-3, TEM-1, SHV-12, SHV-141 | >16 | 128 | 2 | >512 | 0.5 | 512 | 1 |
| K. pneumo JMI 2693 | NDM-1 (Cpase) | >16 | 8 | 0.125 | >512 | 0.5 | 256 | 0.5 |
| K. pneumo JMI 2697 | IMP-4 (Cpase) | 16 | 64 | 0.125 | >512 | >2 | 256 | ≤0.062 |
| K. pneumo JMI 2681 | Oxa-48 (Cpase) | 16 | 64 | 2 | 512 | ≤0.25 | 256 | 0.5 |
| K. pneumo JMI 2699 | VIM-1, CTX-M3 | >16 | 32 | ≤0.031 | >512 | 2 | 128 | ≤0.062 |
| P. aerug JMI 2686 | KPC-2 (Cpase) | 16 | 512 | >2 | 512 | >16 | >512 | >4 |
| P. aerug JMI 149 | Bla+++D+ | >16 | 4 | 0.5 | 256 | >16 | 512 | >4 |
| E. cloacae JMI 36 | P99 | >16 | ≤1 | 0.125 | 128 | 16 | 256 | >4 |
| E. coli JMI 10767 | Wt | 8 | ≤1 | ≤0.031 | ≤1 | ≤0.25 | ≤1 | ≤0.062 |
| E. coli JMI 10768 | CTX-M15 (Weak ESBL) | 8 | ≤1 | ≤0.031 | 8 | ≤0.25 | 8 | ≤0.062 |
| E. coli JMI 10770 | CTX-M15 (Hyper ESBL) | 8 | ≤1 | ≤0.031 | 64 | ≤0.25 | 64 | ≤0.062 |
| E. coli JMI 11103 | CTX-M15 (Intermediate ESBL) | 8 | ≤1 | ≤0.031 | 32 | ≤0.25 | 16 | ≤0.062 |

**Table 7: Synergy of the inhibitor Ex. 7 (4 µg/mL) in combination with antibiotics**

| | | | | | | | | |
|---|---|---|---|---|---|---|---|---|
| 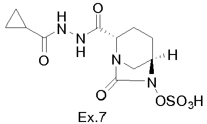 | | | | | | | | |

| **Organism** | **Enzyme** | **Ex. 7 Alone** | **Meropenem** | **Meropenem + Ex. 7 (4 µg/mL)** | **Ceftazidime** | **Ceftazidime + Ex. 7 (4 µg/mL)** | **Aztreonam** | **Aztreonam + Ex. 7 (4 µg/mL)** |
|---|---|---|---|---|---|---|---|---|
| E. coli ATCC 25922 | Wt | >16 | 0.06 | ≤0.0312 | 0.25 | ≤0.25 | 0.25 | ≤0.0625 |
| A. baumanii JMI 2659 | Ges-14 (Cpase) | >16 | 64 | >2 | 512 | >16 | 512 | >4 |
| E.coli JMI 4103 | KPC-2, Tem-1, CMY-Type | 2 | 64 | ≤0.0312 | >512 | ≤0.25 | >512 | ≤0.062 |
| E. coli JMI 4080 | Tem-10 | 4 | ≤1 | ≤0.0312 | 64 | ≤0.25 | 64 | ≤0.062 |
| E. coli JMI 2692 | NDM-1, TEM-1, CTX-M-15 | 8 | 256 | 0.5 | >512 | ≤0.25 | >512 | ≤0.062 |
| E. coli JMI 2671 | VIM-19 (Cpase) | 8 | 64 | 0.5 | 64 | 0.5 | 64 | ≤0.062 |
| E. coli JMI 2665 | CMY-2 (Plasmid Cpase) | 2 | ≤1 | ≤0.0312 | 8 | ≤0.25 | 8 | ≤0.062 |
| K.pneumo JMI 4109 | SHV-1, SHV-12 | 2 | ≤1 | ≤0.0312 | 8 | ≤0.25 | 8 | 0.125 |
| K. pneumo JMI 2673 | CTX-M14 (ESBL) | >16 | ≤1 | ≤0.0312 | 32 | ≤0.25 | 32 | ≤0.062 |
| K. pneumo JMI 2674 | CTX-M15 (ESBL) | >16 | ≤1 | 0.125 | 512 | ≤0.25 | 512 | ≤0.062 |
| K. pneumo JMI 4088 | KPC-3 (Cpase) | >16 | 512 | 1 | >512 | 1 | >512 | 0.125 |
| K. pneumo JMI 4106 | KPC-3, TEM-1, SHV-12, SHV-141 | 16 | 128 | ≤0.0312 | >512 | ≤0.25 | >512 | 0.125 |
| K. pneumo JMI 2693 | NDM-1 (Cpase) | >16 | 8 | 0.25 | >512 | 2 | >512 | 1 |
| K. pneumo JMI 2697 | IMP-4 (Cpase) | >16 | 64 | 2 | >512 | 16 | >512 | ≤0.062 |
| K. pneumo JMI 2681 | Oxa-48 (Cpase) | >16 | 64 | 0.25 | 512 | 0.5 | 512 | 0.25 |
| K. pneumo JMI 2699 | VIM-1, CTX-M3 | 16 | 32 | 0.5 | >512 | 0.5 | >512 | ≤0.062 |
| P. aerug JMI 2686 | KPC-2 (Cpase) | >16 | 512 | >2 | 512 | >16 | 512 | >4 |
| P. aerug JMI 149 | Bla+++D+ | >16 | 4 | >2 | 256 | >16 | 256 | >4 |
| E. cloacae JMI 36 | P99 | >16 | ≤1 | 0.125 | 128 | 0.5 | 128 | 0.125 |
| E. coli JMI 10767 | Wt | 8 | ≤1 | ≤0.0312 | ≤1 | ≤0.25 | ≤1 | ≤0.062 |
| E. coli JMI 10768 | CTX-M15 (Weak ESBL) | 16 | ≤1 | ≤0.0312 | 8 | 2 | 8 | ≤0.062 |
| E. coli JMI 10770 | CTX-M15 (Hyper ESBL) | 8 | ≤1 | ≤0.0312 | 64 | ≤0.25 | 64 | ≤0.062 |
| E. coli JMI 11103 | CTX-M15 (Intermediate ESBL) | 16 | ≤1 | ≤0.0312 | 32 | ≤0.25 | 32 | ≤0.062 |

**Table 8: Synergy of the inhibitor Ex. 8 (4 µg/mL) in combination with antibiotics**

| | | | | | | | | |
|---|---|---|---|---|---|---|---|---|
| 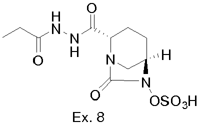 | | | | | | | | |

| **Organism** | **Enzyme** | **Ex. 8 Alone** | **Meropenem** | **Meropenem + Ex. 8 (4 µg/mL)** | **Ceftazidime** | **Ceftazidime + Ex. 8 (4 µg/mL)** | **Aztreonam** | **Aztreonam + Ex. 8 (4 mg/mL)** |
|---|---|---|---|---|---|---|---|---|
| E. coli ATCC 25922 | Wt | 16 | 0.06 | ≤0.0312 | 0.25 | ≤0.25 | 0.125 | ≤0.0625 |
| A. baumanii JMI 2659 | Ges-14 (Cpase) | >16 | 64 | >2 | 512 | >16 | 256 | >4 |
| E.coli JMI 4103 | KPC-2, Tem-1, CMY-Type | 4 | 64 | ≤0.0312 | >512 | ≤0.25 | 256 | ≤0.062 |
| E. coli JMI 4080 | Tem-10 | 4 | ≤1 | ≤0.0312 | 64 | ≤0.25 | 125 | ≤0.062 |
| E. coli JMI 2692 | NDM-1, TEM-1, CTX-M-15 | 4 | 256 | 2 | >512 | ≤0.25 | 256 | ≤0.062 |
| E. coli JMI 2671 | VIM-19 (Cpase) | >16 | 64 | 1 | 64 | 0.5 | 16 | ≤0.062 |
| E. coli JMI 2665 | CMY-2 (Plasmid Cpase) | 4 | ≤1 | ≤0.0312 | 8 | ≤0.25 | 64 | ≤0.062 |
| K.pneumo JMI 4109 | SHV-1, SHV-12 | 4 | ≤1 | ≤0.0312 | 8 | ≤0.25 | ≤1 | ≤0.062 |
| K. pneumo JMI 2673 | CTX-M14 (ESBL) | >16 | ≤1 | ≤0.0312 | 32 | ≤0.25 | 8 | ≤0.062 |
| K. pneumo JMI 2674 | CTX-M15 (ESBL) | >16 | ≤1 | 0.062 | 512 | ≤0.25 | 32 | ≤0.062 |
| K. pneumo JMI 4088 | KPC-3 (Cpase) | >16 | 512 | 0.5 | >512 | 0.5 | 512 | 0.125 |
| K. pneumo JMI 4106 | KPC-3, TEM-1, SHV-12, SHV-141 | >16 | 128 | ≤0.0312 | >512 | ≤0.25 | 512 | ≤0.062 |
| K. pneumo JMI 2693 | NDM-1 (Cpase) | >16 | 8 | 0.125 | >512 | 8 | 256 | 2 |
| K. pneumo JMI 2697 | IMP-4 (Cpase) | >16 | 64 | 2 | >512 | 16 | 256 | ≤0.062 |
| K. pneumo JMI 2681 | Oxa-48 (Cpase) | >16 | 64 | 0.5 | 512 | ≤0.25 | 256 | 0.125 |
| K. pneumo JMI 2699 | VIM-1, CTX-M3 | >16 | 32 | 0.125 | >512 | ≤0.25 | 128 | ≤0.062 |
| P. aerug JMI 2686 | KPC-2 (Cpase) | >16 | 512 | >2 | 512 | >16 | >512 | >4 |
| P. aerug JMI 149 | Bla+++D+ | >16 | 4 | 0.5 | 256 | >16 | 512 | >4 |
| E. cloacae JMI 36 | P99 | >16 | ≤1 | 0.25 | 128 | 0.5 | 256 | 0.125 |
| E. coli JMI 10767 | Wt | >16 | ≤1 | ≤0.0312 | ≤1 | ≤0.25 | ≤1 | ≤0.062 |
| E. coli JMI 10768 | CTX-M15 (Weak ESBL) | >16 | ≤1 | ≤0.0312 | 8 | ≤0.25 | 8 | ≤0.062 |
| E. coli JMI 10770 | CTX-M15 (Hyper ESBL) | >16 | ≤1 | ≤0.0312 | 64 | ≤0.25 | 64 | ≤0.062 |
| E. coli JMI 11103 | CTX-M15 (Intermediate ESBL) | >16 | ≤1 | ≤0.0312 | 32 | ≤0.25 | 16 | ≤0.062 |

**Table 9: Synergy of the inhibitor Ex. 9 (4 µg/mL) in combination with antibiotics**

| | | | | | | | | |
|---|---|---|---|---|---|---|---|---|
| 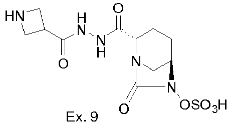 | | | | | | | | |

| **Organism** | **Enzyme** | **Ex. 9 Alone** | **Meropenem** | **Meropenem + Ex. 9 (4 µg/mL)** | **Ceftazidime** | **Ceftazidime + Ex. 9 (4 µg/mL)** | **Aztreonam** | **Aztreonam + Ex. 9 (4 mg/mL)** |
|---|---|---|---|---|---|---|---|---|
| E. coli ATCC 25922 | Wt | >16 | 0.06 | ≤0.03 | 0.25 | ≤0.25 | 0.25 | ≤0.06 |
| A. baumanii JMI 2659 | Ges-14 (Cpase) | >16 | 64 | **2** | 512 | 16 | 512 | >4 |
| E.coli JMI 4103 | KPC-2, Tem-1, CMY-Type | 16 | 64 | 0.06 | >512 | 0.5 | >512 | ≤0.06 |
| E. coli JMI 4080 | Tem-10 | 16 | ≤1 | ≤0.03 | 64 | 0.25 | 64 | ≤0.06 |
| E. coli JMI 2692 | NDM-1, TEM-1, CTX-M-15 | 8 | 256 | 0.125 | >512 | >16 | >512 | 1 |
| E. coli JMI 2671 | VIM-19 (Cpase) | >16 | 64 | 0.125 | 64 | 1 | 64 | ≤0.06 |
| E. coli JMI 2665 | CMY-2 (Plasmid Cpase) | 8 | ≤1 | ≤0.03 | 8 | ≤0.25 | 8 | ≤0.06 |
| K.pneumo JMI 4109 | SHV-1, SHV-12 | 4 | ≤1 | ≤0.03 | 8 | ≤0.25 | 8 | ≤0.06 |
| K. pneumo JMI 2673 | CTX-M14 (ESBL) | >16 | ≤1 | ≤0.03 | 32 | ≤0.25 | 32 | ≤0.06 |
| K. pneumo JMI 2674 | CTX-M15 (ESBL) | >16 | ≤1 | 0.06 | 512 | ≤0.25 | 512 | 0.125 |
| K. pneumo JMI 4088 | KPC-3 (Cpase) | 16 | 512 | 0.06 | >512 | ≤0.25 | >512 | 0.5 |
| K. pneumo JMI 4106 | KPC-3, TEM-1, SHV-12, SHV-141 | 8 | 128 | 0.125 | >512 | ≤0.25 | >512 | 0.5 |
| K. pneumo JMI 2693 | NDM-1 (Cpase) | >16 | 8 | 0.125 | >512 | ≤0.25 | >512 | 0.25 |
| K. pneumo JMI 2697 | IMP-4 (Cpase) | 16 | 64 | 2 | >512 | 8 | >512 | ≤0.06 |
| K. pneumo JMI 2681 | Oxa-48 (Cpase) | >16 | 64 | >2 | 512 | ≤0.25 | 512 | 0.25 |
| K. pneumo JMI 2699 | VIM-1, CTX-M3 | >16 | 32 | 0.5 | >512 | ≤0.25 | >512 | 0.06 |
| P. aerug JMI 2686 | KPC-2 (Cpase) | 16 | 512 | >2 | 512 | >16 | 512 | >4 |
| P. aerug JMI 149 | Bla+++D+ | 16 | 4 | 0.5 | 256 | 16 | 256 | >4 |
| E. cloacae JMI 36 | P99 | >16 | ≤1 | 0.06 | 128 | ≤0.25 | 128 | 0.25 |
| E. coli JMI 10767 | Wt | >16 | ≤1 | ≤0.03 | ≤1 | ≤0.25 | ≤1 | ≤0.06 |
| E. coli JMI 10768 | CTX-M15 (Weak ESBL) | >16 | ≤1 | ≤0.03 | 8 | ≤0.25 | 8 | ≤0.06 |
| E. coli JMI 10770 | CTX-M15 (Hyper ESBL) | >16 | ≤1 | ≤0.03 | 64 | ≤0.25 | 64 | ≤0.06 |
| E. coli JMI 11103 | CTX-M15 (Intermediate ESBL) | >16 | ≤1 | ≤0.03 | 32 | ≤0.25 | 32 | ≤0.06 |

**Table 10: Synergy of inhibitor Ex. 1 (4 µg/mL) in combination with antibiotics against metallo-β-lactamase producing bacteria**

| | | | | | | | | | |
|---|---|---|---|---|---|---|---|---|---|
| 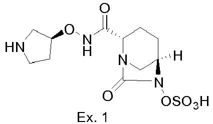 | | | | | | | | | |

| **Organism** | **Isolate No** | **Enzyme** | **Ex.1 alone** | **AZT** | **AZT + Ex. 1** | **CAZ** | **CAZ + Ex. 1** | **MER** | **MER+ Ex.** 1 |
|---|---|---|---|---|---|---|---|---|---|
| Citrobacter freundii | 49469 | NDM-1 | >64 | 256 | 0.5 | >256 | >64 | 16 | 8 |
| Enterobacter aerogenes | 47683 | VIM-12 | >64 | 4 | ≤0.03 | 256 | >64 | 8 | 2 |
| Enterobacter aerogenes | 8397 | VIM-1 | >64 | >256 | 0.5 | >256 | >64 | 64 | 64 |
| Enterobacter cloacae | 1874 | IMP-21 | 4 | ≤0.12 | ≤0.03 | 32 | ≤0.03 | 4 | ≤0.03 |
| Enterobacter cloacae | 1280 | VIM-5 | 4 | 8 | ≤0.03 | 64 | ≤0.03 | 4 | ≤0.03 |
| Enterobacter cloacae | 3686 | IMP-1 | 4 | 32 | ≤0.03 | >256 | ≤0.03 | 8 | 0.25 |
| Enterobacter cloacae | 25 | IMP-4 | 8 | 64 | 0.25 | >256 | 64 | 16 | 2 |
| Enterobacter cloacae | 1471 | VIM-1 | 4 | 64 | ≤0.03 | >256 | ≤0.03 | 2 | ≤0.03 |
| Enterobacter cloacae | 10 | IMP-26 | 64 | 128 | 0.12 | >256 | ≤0.03 | 16 | ≤0.03 |
| Enterobacter cloacae | 53477 | NDM-1 | 4 | 128 | ≤0.03 | >256 | ≤0.03 | 32 | ≤0.03 |
| Enterobacter cloacae | 1068 | VIM-2 | 8 | 128 | 0.06 | 128 | 8 | 2 | ≤0.03 |
| Enterobacter cloacae | 4 | VIM-6 | 64 | 128 | 0.12 | 256 | 32 | 2 | 0.25 |
| Escherichia coli | 13 | IMP-1 | 16 | 16 | ≤0.03 | 256 | ≤0.03 | 4 | ≤0.03 |
| Escherichia coli | 49 | NDM-1 | 4 | 128 | ≤0.03 | >256 | ≤0.03 | 8 | ≤0.03 |
| Escherichia coli | 17 | NDM-1 | 4 | >256 | ≤0.03 | >256 | ≤0.03 | 32 | ≤0.03 |
| Escherichia coli | 53749 | NDM-1 | 4 | >256 | ≤0.03 | >256 | 0.06 | 64 | ≤0.03 |
| Klebsiella oxytoca | 31141 | VIM-23 | >64 | 8 | ≤0.03 | 128 | 2 | 2 | 0.5 |
| Klebsiella oxytoca | 24825 | IMP-26 | >64 | 128 | 0.06 | >256 | >64 | 32 | 4 |
| Klebsiella pneumoniae | 38 | NDM-1 | >64 | 64 | ≤0.03 | >256 | ≤0.03 | 32 | ≤0.03 |
| Klebsiella pneumoniae | 16 | VIM-5 | >64 | 256 | ≤0.03 | >256 | ≤0.03 | 8 | ≤0.03 |
| Providencia stuartii | 26582 | VIM-1 | >64 | 32 | 0.06 | >256 | 32 | 1 | 0.5 |
| Serratia marcescens | 35 | IMP-4 | >64 | ≤0.12 | 0.06 | 64 | 64 | 8 | 4 |
| Serratia marcescens | 36098 | IMP-19 | >64 | >256 | 2 | 128 | 64 | 8 | 8 |

**Table 11: Synergy of inhibitor Ex. 2 (4 µg/mL) in combination with antibiotics against metallo-β-lactamase producing bacteria**

| | | | | | | | | | |
|---|---|---|---|---|---|---|---|---|---|
| 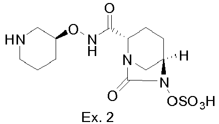 | | | | | | | | | |

| **Organism** | **Isolate No** | **Enzyme** | **Ex. 2 alone** | **AZT** | **AZT + Ex. 2** | **CAZ** | **CAZ+Ex. 2** | **MER** | **MER+ Ex. 2** |
|---|---|---|---|---|---|---|---|---|---|
| Citrobacter freundii | 49469 | NDM-1 | >64 | 256 | 0.06 | >256 | >64 | 16 | 4 |
| Enterobacter aerogenes | 47683 | VIM-12 | >64 | 4 | ≤0.03 | 256 | 32 | 8 | 0.5 |
| Enterobacter aerogenes | 8397 | VIM-1 | >64 | >256 | 0.5 | >256 | >64 | 64 | 64 |
| Enterobacter cloacae | 1874 | IMP-21 | 2 | ≤0.12 | ≤0.03 | 32 | 0.06 | 4 | ≤0.03 |
| Enterobacter cloacae | 1280 | VIM-5 | 4 | 8 | ≤0.03 | 64 | ≤0.03 | 4 | ≤0.03 |
| Enterobacter cloacae | 3686 | IMP-1 | 4 | 32 | ≤0.03 | >256 | ≤0.03 | 8 | ≤0.03 |
| Enterobacter cloacae | 25 | IMP-4 | 4 | 64 | ≤0.03 | >256 | 32 | 16 | 2 |
| Enterobacter cloacae | 1471 | VIM-1 | 4 | 64 | 0.06 | >256 | ≤0.03 | 2 | ≤0.03 |
| Enterobacter cloacae | 10 | IMP-26 | 64 | 128 | ≤0.03 | >256 | ≤0.03 | 16 | ≤0.03 |
| Enterobacter cloacae | 53477 | NDM-1 | 8 | 128 | ≤0.03 | >256 | ≤0.03 | 32 | ≤0.03 |
| Enterobacter cloacae | 1068 | VIM-2 | 4 | 128 | ≤0.03 | 128 | ≤0.03 | 2 | ≤0.03 |
| Enterobacter cloacae | 4 | VIM-6 | 64 | 128 | ≤0.03 | 256 | 0.12 | 2 | 0.25 |
| Escherichia coli | 13 | IMP-1 | 2 | 16 | ≤0.03 | 256 | ≤0.03 | 4 | ≤0.03 |
| Escherichia coli | 49 | NDM-1 | 2 | 128 | ≤0.03 | >256 | ≤0.03 | 8 | ≤0.03 |
| Escherichia coli | 17 | NDM-1 | 2 | >256 | ≤0.03 | >256 | ≤0.03 | 32 | ≤0.03 |
| Escherichia coli | 53749 | NDM-1 | 2 | >256 | ≤0.03 | >256 | ≤0.03 | 64 | ≤0.03 |
| Klebsiella oxytoca | 31141 | VIM-23 | >64 | 8 | ≤0.03 | 128 | 0.5 | 2 | 0.25 |
| Klebsiella oxytoca | 24825 | IMP-26 | >64 | 128 | ≤0.03 | >256 | >64 | 32 | 8 |
| Klebsiella pneumoniae | 38 | NDM-1 | >64 | 64 | ≤0.03 | >256 | ≤0.03 | 32 | ≤0.03 |
| Klebsiella pneumoniae | 16 | VIM-5 | >64 | 256 | ≤0.03 | >256 | ≤0.03 | 8 | ≤0.03 |
| Providencia stuartii | 26582 | VIM-1 | >64 | 32 | ≤0.03 | >256 | 16 | 1 | 0.5 |
| Serratia marcescens | 35 | IMP-4 | >64 | ≤0.12 | ≤0.03 | 64 | 32 | 8 | 2 |
| Serratia marcescens | 36098 | IMP-19 | >64 | >256 | 2 | 128 | 64 | 8 | 8 |

**Table 12: Synergy of inhibitor Ex. 5 (4 µg/mL) in combination with antibiotics against metallo-β-lactamase producing bacteria**

| | | | | | | | | | |
|---|---|---|---|---|---|---|---|---|---|
| 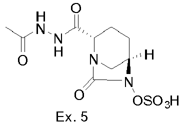 | | | | | | | | | |

| **Organism** | **Isolate No** | **Enzyme** | **Ex. 5 alone** | **AZT** | **AZT + Ex. 5** | **CAZ** | **CAZ+Ex. 5** | **MER** | **MER+ Ex. 5** |
|---|---|---|---|---|---|---|---|---|---|
| Citrobacter freundii | 49469 | NDM-1 | >64 | 256 | ≤0.03 | >256 | >64 | 16 | 2 |
| Enterobacter aerogenes | 47683 | VIM-12 | >64 | 4 | ≤0.03 | 256 | 64 | 8 | 1 |
| Enterobacter aerogenes | 8397 | VIM-1 | >64 | >256 | 0.25 | >256 | >64 | 64 | 64 |
| Enterobacter cloacae | 1874 | IMP-21 | 2 | ≤0.12 | ≤0.03 | 32 | ≤0.03 | 4 | ≤0.03 |
| Enterobacter cloacae | 1280 | VIM-5 | 4 | 8 | ≤0.03 | 64 | ≤0.03 | 4 | ≤0.03 |
| Enterobacter cloacae | 3686 | IMP-1 | 2 | 32 | ≤0.03 | >256 | ≤0.03 | 8 | ≤0.03 |
| Enterobacter cloacae | 25 | IMP-4 | 4 | 64 | ≤0.03 | >256 | 32 | 16 | 2 |
| Enterobacter cloacae | 1471 | VIM-1 | 2 | 64 | ≤0.03 | >256 | ≤0.03 | 2 | ≤0.03 |
| Enterobacter cloacae | 10 | IMP-26 | 64 | 128 | ≤0.03 | >256 | ≤0.03 | 16 | ≤0.03 |
| Enterobacter cloacae | 53477 | NDM-1 | 2 | 128 | ≤0.03 | >256 | ≤0.03 | 32 | ≤0.03 |
| Enterobacter cloacae | 1068 | VIM-2 | 4 | 128 | ≤0.03 | 128 | ≤0.03 | 2 | ≤0.03 |
| Enterobacter cloacae | 4 | VIM-6 | 64 | 128 | ≤0.03 | 256 | 0.25 | 2 | 0.25 |
| Escherichia coli | 13 | IMP-1 | 1 | 16 | ≤0.03 | 256 | ≤0.03 | 4 | ≤0.03 |
| Escherichia coli | 49 | NDM-1 | 2 | 128 | ≤0.03 | >256 | ≤0.03 | 8 | ≤0.03 |
| Escherichia coli | 17 | NDM-1 | 2 | >256 | ≤0.03 | >256 | ≤0.03 | 32 | 0.06 |
| Escherichia coli | 53749 | NDM-1 | 2 | >256 | ≤0.03 | >256 | ≤0.03 | 64 | ≤0.03 |
| Klebsiella oxytoca | 31141 | VIM-23 | >64 | 8 | ≤0.03 | 128 | 2 | 2 | 0.5 |
| Klebsiella oxytoca | 24825 | IMP-26 | >64 | 128 | ≤0.03 | >256 | >64 | 32 | 8 |
| Klebsiella pneumoniae | 38 | NDM-1 | >64 | 64 | ≤0.03 | >256 | ≤0.03 | 32 | ≤0.03 |
| Klebsiella pneumoniae | 16 | VIM-5 | >64 | 256 | ≤0.03 | >256 | ≤0.03 | 8 | ≤0.03 |
| Providencia stuartii | 26582 | VIM-1 | >64 | 32 | ≤0.03 | >256 | 16 | 1 | 0.5 |
| Serratia marcescens | 35 | IMP-4 | >64 | ≤0.12 | ≤0.03 | 64 | 32 | 8 | 2 |
| Serratia marcescens | 36098 | IMP-19 | >64 | >256 | 0.5 | 128 | 64 | 8 | 8 |

### Test for β-lactamase Inhibitory Activity:

The inhibitory activities of compounds herein disclosed against various enzymes were measured by spectrophotometric assay using 490 nM and using nitrocefin as a substrate [J. Antimicrob. Chemother., 28, pp 775-776 (1991)] . The concentration of inhibitor (IC₅₀) which inhibits by 50% the reaction of hydrolysis of nitrocefin by the enzyme is determined. Table 13 shows the results.

**Table 13 Test for β-lactamase Inhibitory Activity**

| Example No. | IC₅₀ (µM) TEM-1 | IC₅₀ (µM) CMY-2 | IC₅₀ (µM) KPC-2 | IC₅₀ (µM) CTX-M-9 |
|---|---|---|---|---|
| Ex. 5 | 0.052 ± 0.004 | 0.008 ± 0.001 | 0.091 ± 0.009 | 0.327 ± 0.020 |

In light of the data described herein, persons of skill in the art would expect that all of the compounds within the scope of formula (I), salts of such compounds, solvates of such compounds and salts thereofwould be effective on their own as antibacterial compounds, and in combination with β-lactam antibiotics.

Efficacy of the β-lactamase inhibitors can be evaluated in combination with ceftazidime (CAZ) aztreonam (AZT), meropenem (MER) and other class of cephalosporins and carbapenems in murine infection models such as septicemia, pneumoniae and thigh infection models (Ref: Andrea Endimiani et. al. Antimicrob. Agents and Chemother Jan 2011, pp-82-85). For murine acute lethal septicemia model, mice were infected by the intraperitoneal injection of the clinical strains resulting in death of the untreated controls within 24-48 hrs. In particular, a fresh predetermined bacterial inoculum of approximately 3.3 x 10⁵ to 3.6 x 10⁵ CFU (colony forming units) in 5% hog gastric mucin grown overnight. Thirty minutes post infection, a single subcutaneous dose of CAZ with and without β-lactamase inhibitor was initiated and the survival ratio monitored for 5 days twice daily. For each strain tested, the dosing regimen used are CAZ alone (doses of 512, 1024 & 2048 mg/kg of body weight) and CAZ plus β-lactamase inhibitor at ratio of 2:1, 4:1, 8:1 & 16:1 (CAZ doses were 4, 8, 16, 32 & 64 mg/kg for each ratio). The median effective dose for 50% (ED₅₀) of animals was determined by a computerized program of Probit analysis. Survival rates stratified for different dosing regimen were also obtained. For experimental pneumoniae model, immunocompromised mice were used and intratracheally infected with *Klebsiella pneumoniae* strains. Mice in this model develop bacteraemia pneumoniae and fatal disease within 2 to 4 days with lung bacterial burden at 16-18 hrs post infection of 10¹¹ to 10¹³ cfu/gm lung. Treatment with CAZ and inhibitor at a ratio of 2/1 & 4/1 demonstrate efficacy with significant 3 to 6 log reduction in lung counts compared to CAZ alone and is relevant to the clinical situation. Human testing of the β-lactamase inhibitor can be conducted in combination with partner antibiotic at a set ratio utilizing standard clinical development practice.

## Claims

1. A compound of formula (I): 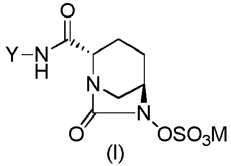 wherein:
M is hydrogen or a pharmaceutically acceptable salt-forming cation;
Y is OR¹ or NR²R³;
R¹ is a radical selected from the group consisting of:
(1) C₃₋₇ cycloalkyl which is fused with a C₅₋₇ membered saturated heterocycle containing at least one heteroatom which is N, the said bicyclic ring optionally substituted;
(2) Bridged bicyclic ring system having optionally one heteroatom which is N, the bicyclic ring system optionally substituted at the free N atom present in the ring; and
(3) C₅₋₇ membered saturated heterocycles fused to a C₃₋₆ membered ring system through a common carbon atom to form a spiro system containing one heteroatom selected from O and N that is present in the spiro ring and the free N atom present in either ring may optionally take a substituent;
R² is hydrogen; and
R³ is a radical selected from any of following groups:
(1) CH₃SO₂-and NH₂SO₂-;
or a pharmaceutically acceptable salt of any such compound, or a pharmaceutically acceptable solvate of any such compound.

2. A compound as claimed in claim 1, wherein Y is OR¹.

3. A compound as claimed in claim 1, wherein Y is NR²R³.

4. A compound as claimed in any one of claims 1 to 3, wherein when R¹ is substituted, said substituent is selected from the following: amino, substituted amino, cyano, amidino, guanidino, and heterocyclylalkyloxy.

5. A compound as claimed in claim 1 or claim 2, which is selected from the following group of compounds: 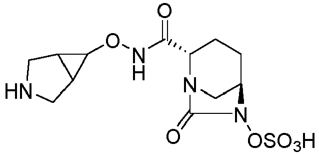 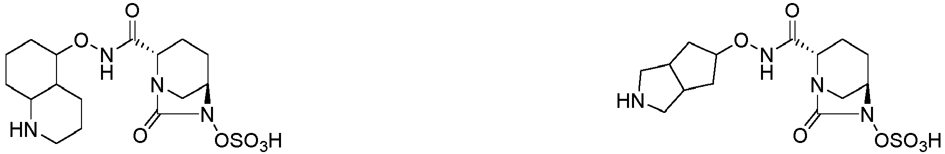 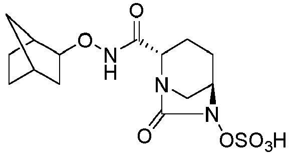 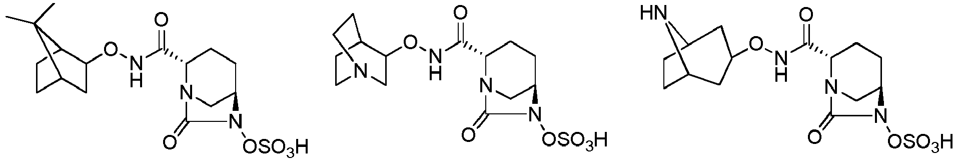 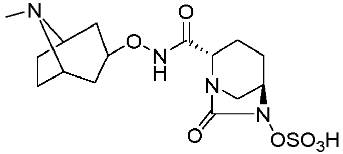 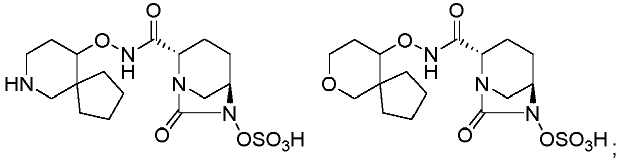 and pharmaceutically acceptable salts of such compounds.

6. A compound of formula (I) as claimed in claim 1 or claim 2, wherein the compound is selected from the group consisting of:
(2*S*,5*R*)-*N*-(3-azabicyclo[3.1.0]hex-6-yloxy)-7-oxo-6-(sulfooxy)-1,6-diazabicyclo[3.2.1]octane-2-carboxamide
(2*S*,5*R*)-*N*-(decahydroquinolin-5-yloxy)-7-oxo-6-(sulfooxy)-1,6-diazabicyclo[3.2.1]octane-2-carboxamide
(2*S*,5*R*)-*N*-(octahydrocyclopenta[c]pyrrol-5-yloxy)-7-oxo-6-(sulfooxy)-1,6-diazabicyclo[3.2.1]octane-2-carboxamide
(2*S*,5*R*)-*N*-(bicyclo[2.2.1]hept-2-yloxy)-7-oxo-6-(sulfooxy)-1,6-diazabicyclo[3.2.1]octane-2-carboxamide
(2*S*,5*R*)-*N*-[(7,7-dimethylbicyclo[2.2.1]hept-2-yl)oxy]-7-oxo-6-(sulfooxy)-1,6-diazabicyclo[3.2.1]octane-2-carboxamide
(2*S*,5*R*)-*N*-(1-azabicyclo[2.2.2]oct-3-yloxy)-7-oxo-6-(sulfooxy)-1,6-diazabicyclo[3.2.1]octane-2-carboxamide
(2*S*,5*R*)-*N*-(8-azabicyclo[3.2.1]oct-3-yloxy)-7-oxo-6-(sulfooxy)-1,6-diazabicyclo[3.2.1]octane-2-carboxamide
(2*S*,5*R*)-*N*-[(8-methyl-8-azabicyclo[3.2.1]oct-3-yl)oxy]-7-oxo-6-(sulfooxy)-1,6-diazabicyclo[3.2.1]octane-2-carboxamide
(2*S*,5*R*)-*N*-(7-azaspiro[4.5]dec-10-yloxy)-7-oxo-6-(sulfooxy)-1,6-diazabicyclo[3.2.1]octane-2-carboxamide
(2*S*,5*R*)-*N*-(7-oxaspiro[4.5]dec-10-yloxy)-7-oxo-6-(sulfooxy)-1,6-diazabicyclo[3.2.1]octane-2-carboxamide.

7. A compound as claimed in claim 1 or claim 3 which is selected from the following group of compounds: 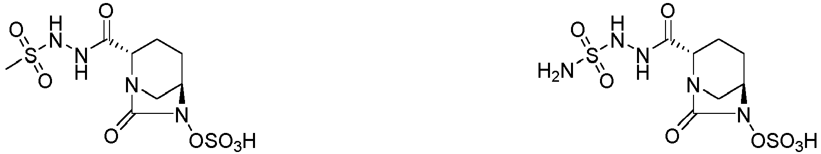 and pharmaceutically acceptable salts of such compounds.

8. A compound of formula (I) as claimed in claim 1 or claim 3, wherein the compound is selected from the group consisting of:
(2*S*,5*R*)-*N'*-(methylsulfonyl)-7-oxo-6-(sulfooxy)-1,6-diazabicyclo[3.2.1]octane-2-carbohydrazide
2-{[(2*S*,5*R*)-7-oxo-6-(sulfooxy)-1,6-diazabicyclo[3.2.1]oct-2-yl]carbonyl}hydrazinesulfonamide.

9. A compound as claimed in any one of claims 1 to 8 for use in therapy.

10. A compound as claimed in any one of claims 1 to 8 for use in treating a bacterial infection in a mammal.

11. A pharmaceutical composition containing as an active ingredient, at least one compound as claimed in any one of claims 1 to 8.

12. A compound as claimed in anyone of claims 1 to 8 for use in a method of treating bacterial infection, said method comprising administering to a mammal in need thereof a combination of (i) an effective amount of said compound and (ii) an effective amount of at least one antibiotic, at least one salt of an antibiotic, at least one hydrate of an antibiotic or at least one prodrug of an antibiotic.

13. A compound for use as claimed in claim 12, wherein said antibiotic is a β-lactam antibiotic.

14. A process for preparing a compound as claimed in claim 1, wherein M = H and Y = OR¹, and wherein the process comprises:
[A] Reacting substituted hydroxylamine (V) with an acid (VI) in presence of a coupling agent selected from the group consisting of EDCI, HOBT-DCC and PyBop to provide an intermediate of formula (VII); 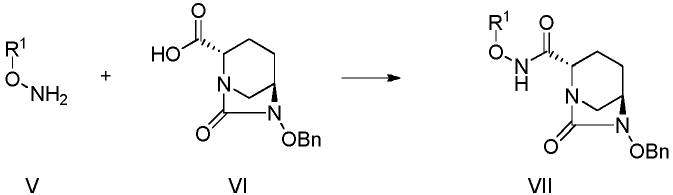
[B] Removing the benzyl protecting group of the intermediate (VII) with a source of hydrogen in presence of Pd catalyst to provide debenzylated product (VIII); 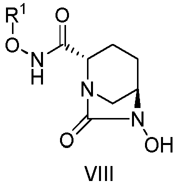
[C] Contacting compound (VIII) with a sulfating agent in the presence of solvent to obtain compound of formula (Ia)

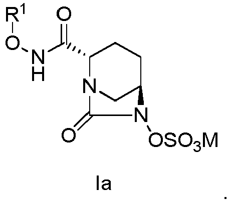

15. A process for preparing a compound as claimed in claim 1, wherein M = H and Y = NR²R³, and wherein the process comprises:
[A] Reacting substituted hydrazine (Va) with an acid (VI) in presence of a coupling agent selected from the group consisting of EDCI or HOBT-DCC and PyBop to provide an intermediate of formula (VIIa), 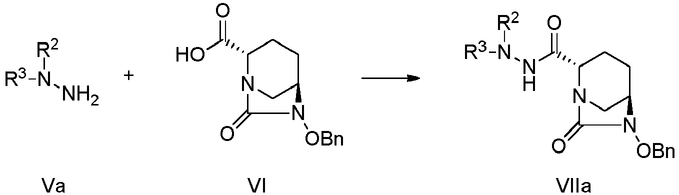
[B] Removing the benzyl protecting group of the intermediate (VIIa) with a source of hydrogen in presence of Pd catalyst to provide the debenzylated product (Villa), 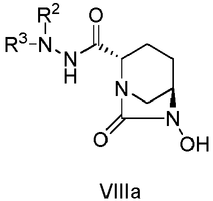
[C] Contacting compound (VIIIa) with a sulfating agent in the presence of solvent to obtain compound of formula (Ib)

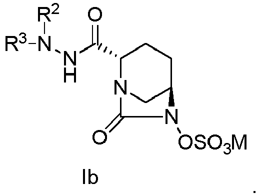

## Patentansprüche

1. Verbindung der Formel (I): 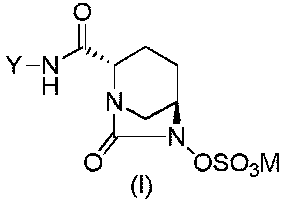 wobei:
M Wasserstoff oder ein pharmazeutisch akzeptables salzbildendes Kation ist;
Y OR¹ oder NR²R³ ist;
R¹ ein Radikal ist, das ausgewählt ist aus der Gruppe, bestehend aus:
(1) C₃₋₇₋Cycloalkyl, das mit einem C₅₋₇-gliedrigen gesättigten Heterozyklus anneliert ist, der mindestens ein Heteroatom enthält, das N ist, wobei der bizyklische Ring wahlweise substituiert ist;
(2) einem verbrückten bizyklischen Ringsystem, das wahlweise ein Heteroatom aufweist, das N ist, wobei das bizyklische Ringsystem wahlweise an dem in dem Ring vorhandenen freien N-Atom substituiert ist; und
(3) C₅₋₇-gliedrigen gesättigten Heterozyklen, die über ein gemeinsames Kohlenstoffatom an ein C₃₋₆-gliedriges Ringsystem anneliert sind, um ein Spirosystem zu bilden, das ein aus O und N ausgewähltes Heteroatom enthält, das in dem Spiroring vorhanden ist, und wobei das in beiden Ringen vorhandene freie N-Atom wahlweise einen Substituenten nehmen kann;
R² Wasserstoff ist; und
R³ ein Radikal ist, das ausgewählt ist aus einer jeglichen von folgenden Gruppen:
(1) CH₃SO₂₋ und NH₂SO₂₋;
oder einem pharmazeutisch akzeptablen Salz einer jeglichen solchen Verbindung oder einem pharmazeutisch akzeptablen Solvat einer jeglichen solchen Verbindung.

2. Verbindung nach Anspruch 1, wobei Y OR¹ ist.

3. Verbindung nach Anspruch 1, wobei Y NR²R³ ist.

4. Verbindung nach einem der Ansprüche 1 bis 3, wobei, wenn R¹ substituiert ist, der Substituent aus den folgenden ausgewählt ist: Amino, substituiertem Amino, Cyano, Amidino, Guanidino und Heterocycloalkyloxy.

5. Verbindung nach Anspruch 1 oder Anspruch 2, die ausgewählt ist aus der folgenden Gruppe von Verbindungen: 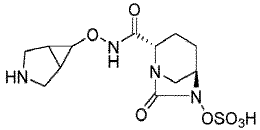 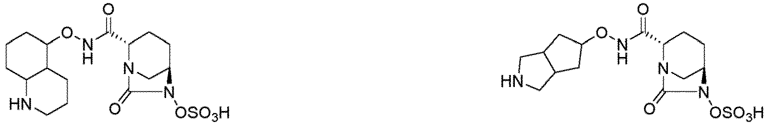 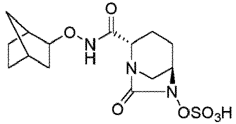 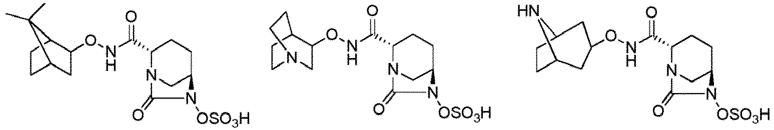 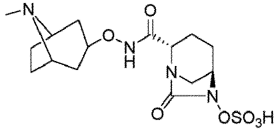 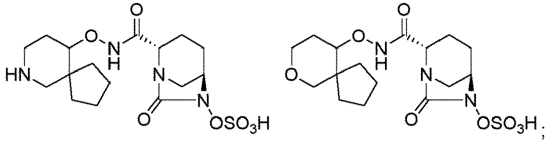 und pharmazeutisch akzeptablen Salzen solcher Verbindungen.

6. Verbindung der Formel (I) nach Anspruch 1 oder Anspruch 2, wobei die Verbindung ausgewählt ist aus der Gruppe, bestehend aus:
(2*S*5*R*)-*N*-(3-Azabicyclo[3-2.1]hex-6-yloxy)-7-oxo-6-(sulfooxy)-1,6-diazabicylo[3.2.1]octan-2-carboxamid
(2*S*5*R*)-*N*-(Decahydrochinolin-5-yloxy)-7-oxo-6-(sulfooxy)-1,6-diazabicylo[3.2.1]octan-2-carboxamid
(2*S*5*R*)-*N*-(Octahydrocyclopenta[*c*]pyrrol-5-yloxy)-7-oxo-6-(sulfooxy)-1,6-diazabicylo[3.2.1]octan-2-carboxamid
(2*S*5*R*)-*N*-(Bicyclo[2.2.1]hept-2-yloxy)-7-oxo-6-(sulfooxy)-1,6-diazabicylo[3.2.1]octan-2-carboxamid
(2*S*5*R*)-*N*-[(7,7-Dimethylbicyclo[2.2.1]hept-2-yl)oxy]-7-oxo-6-(sulfooxy)-1,6-diazabicylo[3.2.1]octan-2-carboxamid
(2*S*5*R*)-*N*-(1-Azabicyclo[2.2.2]oct-3-yloxy)-7-oxo-6-(sulfooxy)-1,6-diazabicylo[3.2.1]octan-2-carboxamid
(2*S*5*R*)-*N*-(8-Azabicyclo[3.2.1]oct-3-yloxy)-7-oxo-6-(sulfooxy)-1,6-diazabicylo[3.2.1]octan-2-carboxamid
(2*S*5*R*)-*N*-[(8-Methyl-8-azabicyclo[3.2.1]oct-3-yl)oxy]-7-oxo-6-(sulfooxy)-1,6-diazabicylo[3.2.1]octan-2-carboxamid
(2*S*5*R*)-*N*-(7-Azaspiro[4.5]dec-10-yloxy)-7-oxo-6-(sulfooxy)-1,6-diazabicylo[3.2.1]octan-2-carboxamid
(2*S*5*R*)-*N*-(7-Oxaspiro[4.5]dec-10-yloxy)-7-oxo-6-(sulfooxy)-1,6-diazabicylo[3.2.1]octan-2-carboxamid.

7. Verbindung nach Anspruch 1 oder Anspruch 3, die ausgewählt ist aus der folgenden Gruppe von Verbindungen: 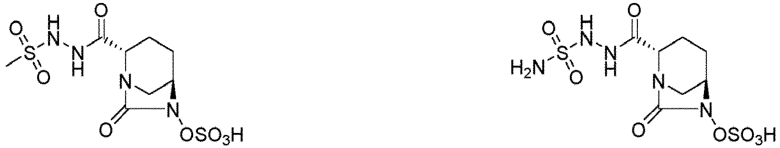 und pharmazeutisch akzeptablen Salzen solcher Verbindungen.

8. Verbindung der Formel (I) nach Anspruch 1 oder Anspruch 3, wobei die Verbindung ausgewählt ist aus der Gruppe, bestehend aus:
(2*S*5*R*)-N'-(Methylsulfonyl)-7-oxo-6-(sulfooxy)-1,6-diazabicyclo[3.2.1]octan-2-carbohydrazid
2-{[(2*S*,5*R*)-7-oxo-(sulfooxy)-1,6-diazabicyclo[3.2.1]oct- 2-yl]carbonyl}hydrazinsulfonamid.

9. Verbindung nach einem der Ansprüche 1 bis 8 zur Verwendung bei der Therapie.

10. Verbindung nach einem der Ansprüche 1 bis 8 zur Verwendung bei der Behandlung einer bakteriellen Infektion bei einem Säugetier.

11. Pharmazeutische Zusammensetzung, enthaltend als Wirkstoff mindestens eine Verbindung nach einem der Ansprüche 1 bis 8.

12. Verbindung nach einem der Ansprüche 1 bis 8 zur Verwendung bei einer Methode zur Behandlung einer bakteriellen Infektion, wobei die Methode das Verabreichen, an ein Säugetier, das eine solche benötigt, einer Kombination (i) einer wirksamen Menge der Verbindung und (ii) einer wirksamen Menge mindestens eines Antibiotikums, mindestens eines Salzes eines Antibiotikums, mindestens eines Hydrats eines Antibiotikums oder mindestens eines Vorläufermedikaments eines Antibiotikums umfasst.

13. Verbindung zur Verwendung nach Anspruch 12, wobei das Antibiotikum ein β-Lactam-Antibiotikum ist.

14. Verfahren zum Herstellen einer Verbindung nach Anspruch 1, wobei M = H und Y = OR¹ und wobei das Verfahren Folgendes umfasst:
[A] Umsetzen von substituiertem Hydroxylamin (V) mit einer Säure (VI) in Gegenwart eines Kopplungsmittels, ausgewählt aus der Gruppe, bestehend aus EDCI, HOBT-DCC und PyBop, um ein Zwischenprodukt der folgenden Formel (VII) bereitzustellen; 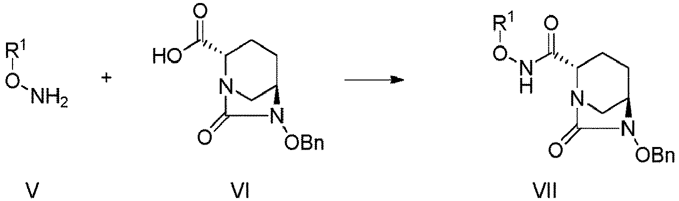
[B] Entfernen der Benzylschutzgruppe des Zwischenprodukts (VII) mit einer Wasserstoffquelle in Gegenwart von Pd-Katalysator, um ein debenzyliertes Produkt (VIII) bereitzustellen; 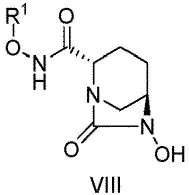
[C] In-Kontakt-Bringen von Verbindung (VIII) mit einem Sulfatierungsmittel in Gegenwart von Lösemittel, um die Verbindung der Formel (Ia) zu erhalten

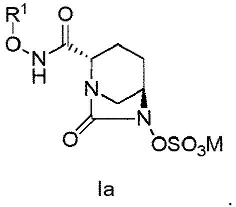

15. Verfahren zum Herstellen einer Verbindung nach Anspruch 1, wobei M = H und Y = NR²R³ und wobei das Verfahren Folgendes umfasst:
[A] Umsetzen von substituiertem Hydrazin (Va) mit einer Säure (VI) in Gegenwart eines Kopplungsmittels, ausgewählt aus der Gruppe, bestehend aus EDCI, HOBT-DCC und PyBop, um ein Zwischenprodukt der folgenden Formel (Vlla) bereitzustellen; 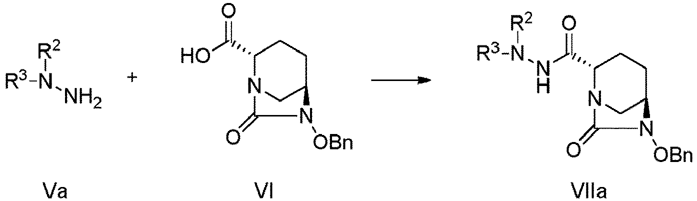
[B] Entfernen der Benzylschutzgruppe des Zwischenprodukts (VIIa) mit einer Wasserstoffquelle in Gegenwart von Pd-Katalysator, um ein debenzyliertes Produkt (VIIIa) bereitzustellen; 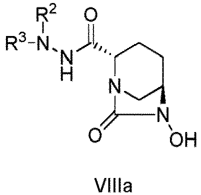
[C] In-Kontakt-Bringen von Verbindung (VIIIa) mit einem Sulfatierungsmittel in Gegenwart von Lösemittel, um die Verbindung der Formel (Ib) zu erhalten

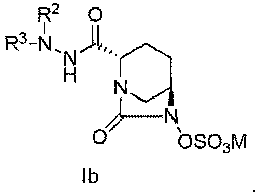

## Revendications

1. Composé de formule (I) : 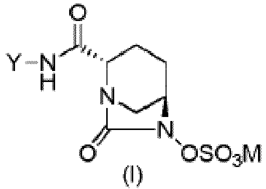 dans laquelle:
M est un hydrogène ou un cation formant un sel pharmaceutiquement acceptable ;
Y est OR¹ ou NR²R³ ;
R¹ est un radical sélectionné dans le groupe consistant en :
(1) un cycloalkyle en C₃₋₇ qui est fusionné à un hétérocycle saturé en C₅₋₇ contenant au moins un hétéroatome qui est N, ledit cycle bicyclique étant facultativement substitué ;
(2) un système de cycle bicyclique ponté présentant facultativement un hétéroatome qui est N, le système de cycle bicyclique étant facultativement substitué sur l'atome N libre présent dans le cycle ; et
(3) les hétérocycles saturés en C₅₋₇ fusionnés à un système cyclique en C₃₋₆ par l'intermédiaire d'un atome de carbone commun pour former un système spiro contenant un hétéroatome sélectionné parmi O et N qui est présent dans le cycle spiro et l'atome N libre présent dans l'un ou l'autre cycle peut facultativement prendre un substituant ;
R² est un hydrogène ; et
R³ est un radical sélectionné parmi l'un quelconque des groupes suivants :
(1) CH₃SO₂- et NH₂SO₂-;
ou un sel pharmaceutiquement acceptable d'un tel composé, ou un solvate pharmaceutiquement acceptable d'un tel composé

2. Composé selon la revendication 1, dans lequel Y est OR¹.

3. Composé selon la revendication 1, dans lequel Y est NR²R³.

4. Composé selon l'une quelconque des revendications 1 à 3, dans lequel quand R¹ est substitué, ledit substituant est sélectionné parmi les suivants : un amino, un amino substitué, un cyano, un amidino, un guanidino, et un hétérocyclylalkyloxy.

5. Composé selon la revendication 1 ou la revendication 2, qui est sélectionné dans le groupe suivant de composés : 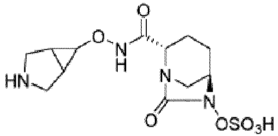 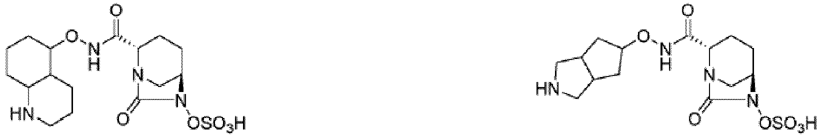 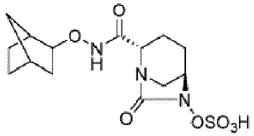 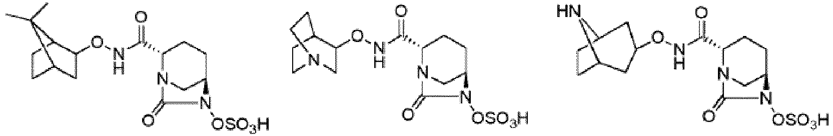 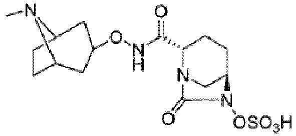 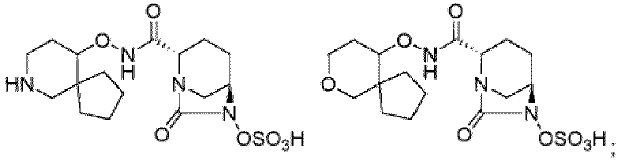 et les sels pharmaceutiquement acceptables de tels composés.

6. Composé de formule (I) selon la revendication 1 ou la revendication 2, dans lequel le composé est sélectionné dans le groupe consistant en :
le (2*S*,5*R*)-*N*-(3-azabicyclo[3.1.0]hex-6-yloxy)-7-oxo-6-(sulfoxy)-1,6-diazabicyclo[3.2.1]octane-2-carboxamide
le (2*S*,5*R*)-*N*-(décahydroquinoléin-5-yloxy)-7-oxo-6-(sulfoxy)-1,6-diazabicyclo[3.2.1]octane-2-carboxamide
le (2*S*,5*R*)-*N*-(octahydrocyclopenta[*c*]pyrrol-5-yloxy)-7-oxo-6-(sulfoxy)-1,6-diazabicyclo[3.2.1]octane-2-carboxamide
le (2*S*,5*R*)-*N*-(bicyclo[2.2.1]hept-2-yloxy)-7-oxo-6-(sulfoxy)-1,6-diazabicyclo[3.2.1]octane-2-carboxamide
le (2*S*,5*R*)-*N*-[(7,7-diméthylbicyclo[2.2.1]hept-2-yl)oxy]-7-oxo-6-(sulfoxy)-1,6-diazabicyclo[3.2.1]octane-2-carboxamide
le (2*S*,5*R*)-*N*-(1-azabicyclo[2.2.2]oct-3-yloxy)-7-oxo-6-(sulfoxy)-1,6-diazabicyclo[3.2.1]octane-2-carboxamide
le (2*S*,5*R*)-*N*-(8-azabicyclo[3.2.1]oct-3-yloxy)-7-oxo-6-(sulfoxy)-1,6-diazabicyclo[3.2.1]octane-2-carboxamide
le (2*S*,5*R*)-*N*-[(8-méthyl-8-azabicyclo[3.2.1]oct-3-yl)oxy]-7-oxo-6-(sulfoxy)-1,6-diazabicyclo[3.2.1]octane-2-carboxamide
le (2*S*,5*R*)-*N*-(7-azaspiro[4.5]déc-10-yloxy)-7-oxo-6-(sulfoxy)-1,6-diazabicyclo[3.2.1]octane-2-carboxamide
le (2*S*,5*R*)-*N*-(7-oxaspiro[4.5]déc-10-yloxy)-7-oxo-6-(sulfoxy)-1,6-diazabicyclo[3.2.1]octane-2-carboxamide

7. Composé selon la revendication 1 ou la revendication 3 qui est sélectionné dans le groupe suivant de composés : 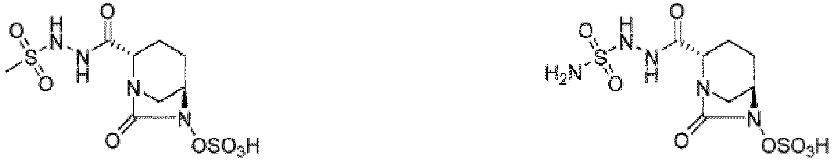 et les sels pharmaceutiquement acceptables de tels composés.

8. Composé de formule (I) selon la revendication 1 ou la revendication 3, dans lequel le composé est sélectionné dans le groupe consistant en :
le (2*S*,5*R*)-*N'*-(méthylsulfonyl)-7-oxo-6-(sulfoxy)-1,6-diazabicyclo[3.2.1]octane-2-carbohydrazide
le 2-{[(2*S*,5*R*)-7-oxo-6-(sulfoxy)-1,6-diazabicyclo[3.2.1]oct-2-yl]carbonyl}hydrazine-sulfonamide.

9. Composé selon l'une quelconque des revendications 1 à 8 pour une utilisation en thérapie.

10. Composé selon l'une quelconque des revendications 1 à 8 pour une utilisation dans le traitement d'une infection bactérienne chez un mammifère.

11. Composition pharmaceutique contenant comme principe actif, au moins un composé selon l'une quelconque des revendications 1 à 8.

12. Composé selon l'une quelconque des revendications 1 à 8 pour une utilisation dans un procédé de traitement d'une infection bactérienne, ledit procédé comprenant l'administration à un mammifère en ayant besoin d'une combinaison (i) d'une quantité efficace dudit composée et (ii) d'une quantité efficace d'au moins un antibiotique, d'au moins un sel d'un antibiotique, d'au moins un hydrate d'un antibiotique ou d'au moins un promédicament d'un antibiotique.

13. Composé pour une utilisation selon la revendication 12, dans lequel ledit antibiotique est un antibiotique β-lactame.

14. Procédé de préparation d'un composé selon la revendication 1, dans lequel M = H et Y = OR¹, et dans lequel le procédé comprend :
[A] la réaction d'une hydroxylamine substituée (V) avec un acide (VI) en présence d'un agent de couplage sélectionné dans le groupe consistant en l'EDCI, l'HOBT-DCC et le PyBop pour fournir un intermédiaire de formule (VII) ; 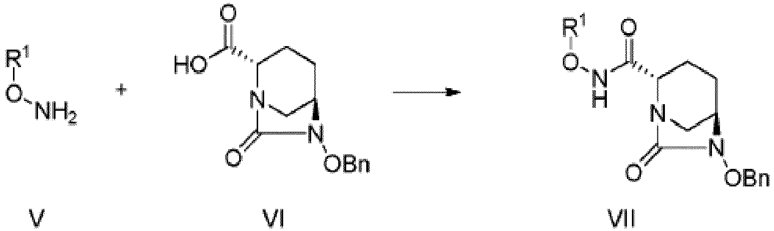
[B] l'élimination du groupe protecteur benzyle de l'intermédiaire (VII) avec une source d'hydrogène en présence d'un catalyseur au Pd pour fournir un produit débenzylé (VIII) ; 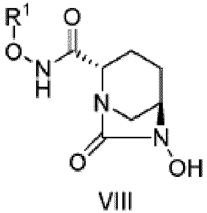
[C] la mise en contact du composé (VIII) avec un agent de sulfatation en présence d'un solvant pour obtenir un composé de formule (la)

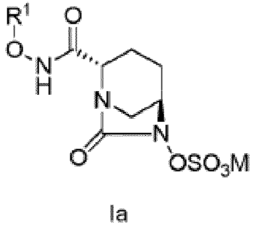

15. Procédé de préparation d'un composé selon la revendication 1, dans lequel M = H et Y = NR²R³, et dans lequel le procédé comprend :
[A] la réaction d'une hydrazine substituée (Va) avec un acide (VI) en présence d'un agent de couplage sélectionné dans le groupe consistant en l'EDCI ou l'HOBT-DCC et le PyBop pour fournir un intermédiaire de formule (VIIa), 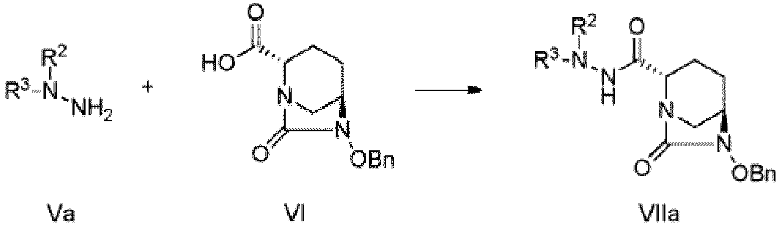
[B] l'élimination du groupe protecteur benzyle de l'intermédiaire (VIIa) avec une source d'hydrogène en présence d'un catalyseur au Pd pour fournir le produit débenzylé (VIIIa) ; 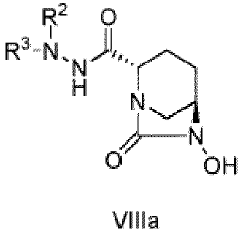
[C] la mise en contact du composé (VIIIa) avec un agent de sulfatation en présence d'un solvant pour obtenir un composé de formule (Ib)
